**Europäisches Patentamt**

**European Patent Office**

**Office européen des brevets**

(19)

(11) **EP 1 452 538 A1**

(12) # EUROPEAN PATENT APPLICATION

(43) Date of publication:
**01.09.2004 Bulletin 2004/36**

(51) Int Cl.⁷: **C07K 1/04**, C07K 17/06

(21) Application number: **04250807.7**

(22) Date of filing: **16.02.2004**

(84) Designated Contracting States:
**AT BE BG CH CY CZ DE DK EE ES FI FR GB GR HU IE IT LI LU MC NL PT RO SE SI SK TR**
Designated Extension States:
**AL HR LT LV MK**

(30) Priority: **25.02.2003 US 449895 P**

(71) Applicant: **ROHM AND HAAS COMPANY**
**Philadelphia, Pennsylvania 19106-2399 (US)**

(72) Inventors:
• **Bohling, James Charles**
  **Lansdale, Pennsylvania 19446 (US)**
• **Kinzey, Marlin Kenneth**
  **Philadelphia, Pennsylvania 19147 (US)**
• **Maikner, John Joseph**
  **Zionsville, Pennsylvania 18092 (US)**
• **Zabrodski, William Joseph**
  **Lansdale, Pennsylvania 19446 (US)**
• **Ziarno, Witold Andrew**
  **Chicago, Illinois 60632 (US)**

(74) Representative: **Kent, Venetia Katherine et al**
  **Rohm and Haas (UK) Ltd.,**
  **European Patent Department,**
  **28th Floor,**
  **City Point,**
  **One Ropemaker Street**
  **London EC2Y 9HS (GB)**

(54) **Method of manufacturing T-20 and T-1249 peptides at commercial scale, and T-20 and T-1249 compositions related thereto**

(57)  The present invention relates to an improved process for making commercial quantities of a T-20 or a T-1249 composition, or a fragment of a T-20 or a T-1249 composition. The improvement includes using a low void space resin and/or a surface functionalized resin optionally loaded with an amino acid or amino acid derivative to create one or more T-20 or T-1249 fragments.

**EP 1 452 538 A1**

## Description

This invention relates to methods of manufacturing T-20, T-1249, T-20 like peptides, and T-1249 like peptides using functionalized low void space polymeric resins useful as supports in solid phase synthesis.

There exists a significant need in the art for methods of manufacturing the above mentioned peptides at commercial scale. While various techniques are known to work at lab scale, these techniques have a variety of drawbacks. These drawbacks include high cycle times, wasteful use of expensive reagents, poor yields at scale, poor loading efficiencies at scale, use of high volumes of expensive solvents at scale, the need to recouple, and poor purities at scale. Moreover, attempts to reduce cycle times or excesses of expensive reagents have lead to lower product yields and purity and required increased recoupling. There have been attempts to recycle conventional CTC resins found in the art (Harre et al. Reactive and Functional Polymers 41 (1999) 111-114). However, these attempts failed since there were serious problems with resins of the prior art. The resins of the prior art suffered from physical degradation of the polymer.

It is an object of the invention to solve these and other problems facing the art, and to provide a method by which commercially useful quantities of T-20, and T-1249 can be manfuctured.

## SUMMARY OF THE INVENTION

In one aspect the present invention relates to an improved process for making a T-20 or a T-1249 composition, or a fragment of a T-20 or a T-1249 composition. The improvement includes using a low void space resin optionally loaded with an amino acid or amino acid derivative to create one or more T-20 or T-1249 fragments.

In another aspect, the process uses about 1.5 equivalents of the amino acid per equivalent of growing peptide chain.

In yet a further aspect, the improved process for making a T-20 or a T-1249 composition, or a fragment of a T-20 or a T-1249 composition includes providing a cycle time in the range of about 15 minutes to about 30 minutes, and obtaining a load efficiency of amino acid that is greater than that obtained by conventional methods.

In yet a further aspect, the process includes recycling the low void space resin.

In yet another aspect the invention provides for preparing a T-20 or T-1249 fragment having greater than about 10 or about 15 amino acids, in which the process is free of or substantially free of recouples and uses substantially lower quantities of reagents.

In yet another variant, the invention provides a T-20 or T-1249 composition, in which one or more fragments of T-20 or T-1249 are made by the processes described herein.

In yet another aspect, the improved process includes using a resin having functionality biased toward the surface of the resin. In yet another aspect, the improved process includes using a low void space resin having functionality biased toward the surface of the low void space resin.

These and other objects of the invention are described in the remaining portions of the specification, including but not limited to the detailed description of the invention.

## DETAILED DESCRIPTION OF THE INVENTION

The present invention relates to an improved process for making a T-20 or a T-1249 composition, or a fragment of a T-20 or a T-1249 composition. The improvement includes using a low void space resin and/or a resin having surface biased functionalization. The resin is optionally loaded with an amino acid or amino acid derivative to create one or more T-20 or T-1249 fragments.

An exemplary "amino acid" that can be used with the resins described in the present invention, and loaded on the resin as described herein, is a compound represented by NH.sub.2--CHR--COOH, wherein R is H, an aliphatic group, a substituted aliphatic group, an aromatic group or a substituted aromatic group. A "naturally-occurring amino acid" is found in nature. Examples include alanine, valine, leucine, isoleucine, aspartic acid, glutamic acid, serine, threonine, glutamine, asparagine, arginine, lysine, ornithine, proline, hydroxyproline, phenylalanine, tyrosine, tryptophan, cysteine, methionine and histidine. R is the side-chain of the amino acid. Examples of naturally occurring amino acid side-chains include methyl (alanine), isopropyl (valine), sec-butyl (isoleucine), --CH.sub.2CH(--CH).sub.2 (leucine), benzyl (phenylalanine), p-hydroxybenzyl (tyrosine), --CH.sub.2OH (serine), CHOHCH.sub.3 (threonine), --CH.sub.2-3-indoyl (tryptophan), --CH.sub.2COOH (aspartic acid), CH.sub.2CH.sub.2COOH (glutamic acid), --CH.sub.2C(O)NH.sub.2 (asparagine), --CH.sub.2CH.sub.2C(O)NH.sub.2 (glutamine), --CH.sub.SSH, (cysteine), --CH.sub.2CH.sub.2SCH.sub.3 (methionine), --(CH.sub.2).sub.4NH.sub.2 (lysine), -- (CH.sub.2).sub.3NH.sub.2 (omithine), -[(CH).sub.2].sub.4NHC(.- dbd.NH)NH.sub.2 (arginine) and --CH.sub.2-3-imidazoyl (histidine). The side-chains of alanine, valine, leucine and isoleucine are aliphatic, i.e., contain only carbon and hydrogen, and are each referred to herein as "the aliphatic side chain of a naturally occurring amino acid.

The side chains of other naturally-occurring amino acids that can be used in the present invention include a heteroatom-containing functional group, e.g., an alcohol (serine, tyrosine, hydroxyproline and threonine), an amine (lysine, omithine, histidine and arginine), a thiol (cysteine) or a carboxylic acid (aspartic acid and glutamic acid). When the heteroatom-containing functional group is modified to include a protecting group, the side-chain is referred to as the "protected side-chain" of an amino acid.

The selection of a suitable protecting group depends upon the functional group being protected, the conditions to which the protecting group is being exposed and to other functional groups which may be present in the molecule. Suitable protecting groups for the functional groups discussed above are described in Greene and Wuts, "Protective Groups in Organic Synthesis", John Wiley & Sons (1991), the entire teachings of which are incorporated into this application by reference as if fully set forth herein. The skilled artisan can select, using no more than routine experimentation, suitable protecting groups for use in the disclosed synthesis, including protecting groups other than those described below, as well as conditions for applying and removing the protecting groups.

Examples of suitable alcohol protecting groups include benzyl, allyl, trimethylsilyl, tert-butyldimethylsilyl, acetate, and the like. Examples of suitable amino protecting groups include benzyloxycarbonyl, tert-butoxycarbonyl, tert-butyl, benzyl and fluorenylmethyloxycarbonyl (Fmoc). Tert-butoxycarbonyl is an amine protecting group. Examples of suitable carboxylic acid protecting groups include tert-butyl, trityl, methyl, methoxylmethyl, trimethylsilyl, benzyloxymethyl, tert-butyldimethylsilyl and the like. Tert-butyl is a carboxylic acid protecting group. Examples of suitable thiol protecting groups include S-benzyl, S-tert-butyl, S-acetyl, S-methoxymethyl, S-trity land the like.

Lysine, aspartate and threonine are examples of amino acid side-chains that are preferably protected in one variant of the invention. Aliphatic groups include straight chained, branched $C_1$-$C_8$, or cyclic $C_3$-$C_8$ hydrocarbons which are completely saturated or which contain one or more units of unsaturation. In one example, an aliphatic group is a C1-C4 alkyl group. Aromatic groups include carbocyclic aromatic groups such as phenyl, 1-naphthyl, 2-naphthyl, 1-anthracyl and 2-anthracyl, and heterocyclic aromatic groups such as N-imidazolyl, 2-imidazole, 2-thienyl, 3-thienyl, 2-furanyl, 3-furanyl, 2-pyridyl, 3-pyridyl, 4-pyridyl, 2-pyrimidy, 4-pyrimidyl, 2-pyranyl, 3-pyranyl, 3-pyrazolyl, 4-pyrazolyl, 5-pyrazolyl, 2-pyrazinyl, 2-thiazole, 4-thiazole, 5-thiazole, 2-oxazolyl, 4-oxazolyl and 5-oxazolyl.

Aromatic groups also include fused polycyclic aromatic ring systems in which a carbocyclic aromatic ring or heteroaryl ring is fused to one or more other heteroaryl rings. Examples include 2-benzothienyl, 3-benzothienyl, 2-benzofuranyl, 3-benzofuranyl, 2-indolyl, 3-indolyl, 2-quinolinyl, 3-quinolinyl, 2-benzothiazole, 2-benzooxazole, 2-benzimidazole, 2-quinolinyl, 3-quinolinyl, 1-isoquinolinyl, 3-quinolinyl, 1-isoindolyl, 3-isoindolyl, and acridintyl.

Suitable substituents for an aryl group and aliphatic group are those which are compatible with the disclosed reactions, i.e., do not significantly reduce the yield of the reactions and do not cause a significant amount of side reactions. Suitable substituents generally include aliphatic groups, substituted aliphatic groups, aryl groups, halogens, halogenated alkyl groups (e.g., trihalomethyl), nitro, nitrile, --CONHR, --CON(R)$_2$, --OR, --SR, --S(O)R, --S(O)$_2$R, wherein each R is independently an aliphatic group, or an aryl group. Although certain functional groups may not be compatible with one or more of the disclosed reactions, these functional groups may be present in a protected form. The protecting group can then be removed to regenerate the original functional group. Skilled artisan will be able to select, using no more than routine experimentation, protecting groups which are compatible with the disclosed reactions.

A peptide mimetic, or component thereof, can also be used in the present invention, loaded onto a resin as described herein, or created by the process described herein. A peptide mimetic is a compound which has sufficient structural similarity to a peptide so that the desirable properties of the peptide are retained by the mimetic. For example, peptide mimetics used as protease inhibitors for treating HIV infection, are disclosed in Tung, et al., WO 94/05639, Vazquez, et al., WO 94/04491, Vazquez, et al., WO 94/10134 and Vaquez, et al., WO 94/04493. The entire relevant teachings of these publications are incorporated herein by reference. To be useful as a drug, a peptide mimetic should retain the biological activity of a peptide, but also have one or more properties which are improved compared with the peptide which is being mimicked. For example, some peptide mimetics are resistant to hydrolysis or to degradation in vivo. One strategy for preparing a peptide mimetic is to replace one or more amino acid residues in a peptide with a group which is structurally related to the amino acid residue(s) being replaced and which can form peptide bonds. The development of new amino acid derivatives which can be used to replace amino acid residues in peptides will advance the development of new peptide mimetic drugs.

Exemplary peptide mimetics are described in United States Patent Application Serial No. 20020188135 by Gabriel, Richard L. et al. filed on December 12, 2002 entitled, "Amino acid derivatives and methods of making the same." This patent application is incorporated by reference herein as if fully set forth. Also useful in the present invention are physiologically acceptable salts of these compounds. Salts of compounds containing an amine or other basic group can be obtained, for example, by reacting with a suitable organic or inorganic acid, such as hydrogen chloride, hydrogen bromide, acetic acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium and the like.

The present invention is also useful in the creation and manufacture of therapeutic agents and biologically active substances that have one or more peptides, peptide derivatives, or peptide mimetics as building blocks or constituents thereof. Compounds or fragments of a compound which are terminated with an ester functionality can also be created by the present invention. The therapeutic agent that can be manufactured or created using the invention can vary widely with the purpose for the composition. The agent(s) may be described as a single entity or a combination of entities. The delivery system is designed to be used with therapeutic agents having high water-solubility as well as with those having low water-solubility to produce a delivery system that has controlled release rates. The terms "therapeutic agent" and "biologically active substance" include without limitation, medicaments; vitamins; mineral supplements; substances used for the treatment, prevention, diagnosis

**Resin Manufacture Example**

One aspect of the invention includes an improved process for making a T-20 or T-1249 composition, a fragment of a T-20 or T-1249 composition using a chlorotrityl chloride linker-resin. The CTC resin is a resin having functionality biased toward the surface of the resin. The surface bias of the functionality places the linker group closer to the surface of the resin where it can provide better accessibility to the growing peptide chains. The surface bias is created by loading the linker to the resin under controlled swell conditions. By contacting the functionalizing reagents with the resin in a non fully swollen state, the linker is located in more sterically accessible areas of the bead. This process is described in more detail in U.S. Provisional Patent Application, by Bohling et al., filed 8/16/02, Serial No.: 60/404,044, entitled: RESIN FOR SOLID PHASE SYNTHESIS (DN A01407), incorporated by reference herein as if fully set forth. The resin is optionally loaded with an amino acid or amino acid derivative, to create one or more T-20 or T-1249 fragments.

By way of example, the present invention uses a crosslinked polymer bead which, when: (i) functionalized with a 2-chlorotrityl chloride group; (ii) coupled with Leu to 0.65 mmol/g; and (iii) coupled with Glu(t-Bu); allows coupling of FMOC-Lys(BOC)-OH at an amount of 1.5 equivalents in the presence of 1.5 equivalents of HOBT, 1.5 equivalents of DIEA and 1.5 equivalents of HBTU, to be completed, as determined by the Kaiser test, in no more than 35 minutes.

The present invention uses a functionalized crosslinked polymer bead produced by a method comprising steps of: (a) swelling the bead in a first solvent or solvent mixture to a volume from 200% to 310% of its volume when dry; and (b) contacting the bead with a functionalizing reagent in a second solvent or solvent mixture capable of swelling the bead to a volume from 200% to 310% of its volume when dry.

The present invention uses a functionalized crosslinked polymer bead produced by contacting the bead at 100% to 200% of its volume when dry with a functionalizing reagent in a solvent or solvent mixture capable of swelling the bead to a volume from 200% to 400% of its volume when dry. The functionalized resin may swell to as much as 700% by the end of the reaction.

Percentages are weight percentages, unless specified otherwise. As used herein the term "(meth)acrylic" refers to acrylic or methacrylic. The term "vinyl monomer" refers to a monomer suitable for addition polymerization and containing a single polymerizable carbon-carbon double bond. The term "styrene polymer" indicates a copolymer polymerized from a vinyl monomer or mixture of vinyl monomers containing at least 50 weight percent, based on the total monomer weight, of styrene monomer, along with at least one crosslinker. Preferably a styrene polymer is made from a mixture of monomers that is at least 75% styrene, more preferably at least 90% styrene, and most preferably from a mixture of monomers that consists essentially of styrene and at least one vinylaromatic crosslinker. The polymeric bead used as a starting material in this invention contains monomer residues from at least one monomer having one copolymerizable carbon-carbon double bond and at least one crosslinker. The monomer residues derived from the crosslinker are from 0.5 mole percent to 1.5 mole percent based on the total of all monomer residues. Preferably the amount of crosslinker is from 0.7 to 1.3 mole percent, more preferably from 0.7 to 1.2 mole percent, and most preferably from 0.8 to 1.2 mole percent.

A polymeric bead used as a starting material in the present invention preferably is a spherical copolymer bead having a particle diameter no greater than 200 microns (μm), preferably no greater than 170 μm, more preferably no greater than 150 μm, more preferably no greater than 125 μm, and most preferably no greater than 100 μm. Preferably, the bead has no void spaces having a diameter greater than 3 μm, more preferably no void spaces having a diameter greater than 2 μm, and most preferably no void spaces having a diameter greater than 1 μm. Typically, void spaces are readily apparent upon surface examination of the bead by a technique such as light microscopy.

The polymeric bead used as a starting material in the present invention preferably is produced by a suspension polymerization. A typical bead preparation, for example, may include preparation of a continuous aqueous phase solution containing typical suspension aids, for example, dispersants, protective colloids and buffers. Preferably, to aid in production of relatively small beads, a surfactant is included in the aqueous solution, preferably a sodium alkyl sulfate surfactant, and vigorous agitation is maintained during the polymerization process. The aqueous solution is combined with a monomer mixture containing at least one vinyl monomer, at least one crosslinker and at least one free-radical initiator. Preferably, the total initiator level is from 0.25 mole percent to 2 mole %, based on the total monomer charge, preferably from 0.4 mole percent to 1.5 mole percent, more preferably from 0.4 mole percent to 1 mole percent, and most preferably from 0.5 mole percent to 0.8 mole percent. The mixture of monomers is then polymerized at elevated temperature. Preferably, the polymerization is continued for a time sufficient to reduce the unreacted vinyl monomer content to less than 1% of the starting amount. The resulting bead is then isolated by conventional means, such as dewatering, washing with an aprotic organic solvent, and drying.

Crosslinkers are monomers having 2 or more copolymerizable carbon-carbon double bonds per molecule, such as: divinylbenzene, divinyltoluene, divinylxylene, trivinylbenzene, trivinylcyclohexane, divinylnaphthalene, trivinylnaphthalene, diethyleneglycol divinylether, ethyleneglycol dimethacrylate, polyethyleneglycol dimethacrylate, triethyleneglycol dimethacrylate, trimethylolpropane trimethacrylate, allyl methacrylate, 1,5-hexadiene, 1,7-octadiene or 1,4-bis(4-vinylphenoxy)butane; it is understood that any of the various positional isomers of each of the aforementioned crosslinkers is suitable. Preferred crosslinkers are divinylbenzene, divinyltoluene, trivinylbenzene or 1,4-bis(4-vinylphenoxy)butane. The most preferred crosslinker is divinylbenzene.

Suitable monounsaturated vinylaromatic monomers that may be used in the preparation of the bead used as a starting material in the present invention include, for example, styrene, $\alpha$-methylstyrene, $(C_1-C_4)$alkyl-substituted styrenes and vinylnaphthalene; preferably one or more monounsaturated vinylaromatic monomer is selected from the group consisting of styrene and $(C_1-C_4)$alkyl-substituted styrenes. Included among the suitable $(C_1-C_4)$alkyl-substituted styrenes are, for example, ethylvinylbenzenes, vinyltoluenes, diethylstyrenes, ethylmethylstyrenes, dimethylstyrenes and isomers of vinylbenzyl chloride; it is understood that any of the various positional isomers of each of the aforementioned vinylaromatic monomers is suitable.

Optionally, non-aromatic vinyl monomers, such as aliphatic unsaturated monomers, for example, acrylonitrile, glycidyl methacrylate, (meth)acrylic acids and amides or $C_1-C_6$ alkyl esters of (meth)acrylic acids may also be used in addition to the vinylaromatic monomer. When used, the non-aromatic vinyl monomers typically comprise as polymerized units, from zero to 20%, preferably from zero to 10%, and more preferably from zero to 5% of the copolymer, based on the total monomer weight used to form the copolymer.

Preferred vinyl monomers are the vinylaromatic monomers; more preferably styrene, isomers of vinylbenzyl chloride, and $\alpha$-methylstyrene. The most preferred vinyl monomer is styrene.

Polymerization initiators useful in the present invention include monomer-soluble initiators such as peroxides, hydroperoxides, peroxyesters and related initiators; for example benzoyl peroxide, tert-butyl hydroperoxide, cumene peroxide, tetralin peroxide, acetyl peroxide, caproyl peroxide, tert-butyl peroctoate (also known as tert-butylperoxy-2-ethylhexanoate), *tert-amyl* peroctoate, tert-butyl perbenzoate, tert-butyl diperphthalate, dicyclohexyl peroxydicarbonate, di(4-tert-butylcyclohexyl)peroxydicarbonate and methyl ethyl ketone peroxide. Also useful are azo initiators such as azodiisobutyronitrile, azodiisobutyramide, 2,2'-azo-bis(2,4-dimethylvaleronitrile), azo-bis($\alpha$,-methyl-butyronitrile) and dimethyl-, diethyl- or dibutyl azo-bis(methylvalerate). Preferred peroxide initiators are diacyl peroxides, such as benzoyl peroxide, and peroxyesters, such as tert-butyl peroctoate and tert-butyl perbenzoate.

Dispersants and suspending agents useful in the present invention are nonionic surfactants having a hydroxyalkylcellulose backbone, a hydrophobic alkyl side chain containing from 1 to 24 carbon atoms, and an average of from 1 to 8, preferably from 1 to 5, ethylene oxide groups substituting each repeating unit of the hydroxyalkyl-cellulose backbone, the alkyl side chains being present at a level of 0.1 to 10 alkyl groups per 100 repeating units in the hydroxyalkylcellulose backbone. The alkyl group in the hydroxyalkylcellulose may contain from 1 to 24 carbons, and may be linear, branched or cyclic. More preferred is a hydroxyethylcellulose containing from 0.1 to 10 $(C_{16})$alkyl side chains per 100 anhydroglucose units and from about 2.5 to 4 ethylene oxide groups substituting each anhydroglucose unit. Typical use levels of dispersants are from about 0.01 to about 4%, based upon the total aqueous-phase weight. One example of a useful dispersant in Culminal MHEC 8000, commercially available from Hercules of Wilmington, Delaware.

Optionally, the preparation of the beads may include an enzyme treatment to cleanse the polymer surface of residues of dispersants and suspending agents used during the polymerization. The enzyme treatment typically involves contacting the polymeric phase with the enzymatic material (selected from one or more of cellulose-decomposing enzyme and proteolytic enzyme) during polymerization, following polymerization or after isolation of the polymer. Japanese Patent Applications No. 61-141704 and No. 57-98504 may be consulted for further general and specific details on the use of enzymes during the preparation of polymer resins. Suitable enzymes include, for example, cellulose-decomposing enzymes, such as $\beta$-1,4-glucan-4-glucano-hydrase, $\beta$-1,4-glucan-4-glucanhydrolase, $\beta$-1,4-glucan-4-glucohydrase and $\beta$-1,4-glucan-4-cellobiohydrase, for cellulose-based dispersant systems; and proteolytic enzymes, such as urokinase, elastase and enterokinase, for gelatin-based dispersant systems. Typically, the amount of enzyme used relative to the polymer is from 2 to 35%, preferably from 5 to 25% and more preferably from 10 to 20%, based on total weight of polymer.

In the method of the present invention, the swelling of the crosslinked polymeric beads is controlled so that the bead is partially swelled during functionalization. Without wishing to be bound by theory, the effect of functionalizing a partially swollen bead is to limit the location of the attached functional groups to a region relatively close to the surface of the bead. Preferably, when the functionalization occurs, the bead is swollen to at least 200% of its volume when dry, more preferably at least 210%, more preferably at least 220%, more preferably at least 230%, and most preferably at least 240%. Preferably, the bead is swollen to no more than 310% of its volume when dry, more preferably no more than 300%, more preferably no more than 290%, and most preferably no more than 280%. There are different means for accomplishing the desired degree of swelling during functionalization.

In one embodiment of the invention, a bead which is not pre-swollen (i.e., at 100% of its volume when dry), or which is pre-swollen to no more than 200% of its volume when dry, is contacted with a functionalizing reagent in a solvent or solvent mixture capable of swelling the bead to at least 200% of its volume when dry, more preferably at least 210%, more preferably at least 220%, more preferably at least 230%, and most preferably at least 240%. Preferably, the solvent or solvent mixture is capable of swelling the bead to no more than 400% of its volume when dry, more preferably no more than 370%, more preferably no more than 340%, and most preferably no more than 320%. Preferably, the bead is pre-swollen to no more than 150%, more preferably no more than 100%, more preferably no more than 80%, more preferably no more than 60%, and most preferably no more than 40%. In one embodiment, the bead is used in its dry state without pre-swelling. The resin may swell to greater than 700% by the completion of the reaction.

In another embodiment of the invention, the bead is pre-swollen in a solvent or solvent mixture which swells the bead to at least 200% of its volume when dry, more preferably at least 210%, more preferably at least 220%, more preferably at least 230%, and most preferably at least 240%. Preferably, the bead is swollen to no more than 310% of its volume when dry, more preferably no more than 300%, more preferably no more than 290%, and most preferably no more than 280%. After pre-swelling, the bead is contacted with a functionalizing reagent in a solvent or solvent mixture capable of swelling the bead within the aforementioned limits. Most preferably, the solvents or solvent mixtures used for pre-swelling and functionalization are the same.

A functionalizing reagent is one which covalently attaches a functional group to the polymer comprising the bead. Further elaboration of the functional group may be necessary to maximize the utility of the bead as a support for solid phase synthesis. However, the initial attachment of the functional group determines the region of the bead which is functionalized and thus tends to control the ability of the bead to react with substrates for solid phase synthesis and to allow recovery of the synthetic product. For styrene polymers, the functionalization typically is a Friedel-Crafts substitution on the aromatic ring, preferably an acylation, bromination, or halomethylation. Subsequent elaboration of the initial functional group typically is done. For example, acylation by aroyl halides often is followed by addition of an aryl lithium to the carbonyl group of the product to produce a triaryl carbinol functional group, which then is halogenated to produce a trityl halide functional group. In one preferred embodiment of the invention, 2-chlorobenzoyl chloride, followed by phenyllithium, and then thionyl chloride, produces a 2-chlorotrityl chloride functional group. Bromination typically is followed by treatment with an alkyl lithium reagent and reaction of the aryl lithium product with a variety of reagents to produce different functional groups. Halomethyl groups also may react with a variety of reagents to produce different functional groups.

Solvents capable of partially swelling the bead include, for example, $C_1$-$C_6$ nitroalkanes, and mixtures of relatively non-swelling solvents such as alkanes with nitrobenzene or chlorinated hydrocarbons. For functionalization using Friedel-Crafts chemistry, $C_3$-$C_6$ nitroalkanes, and mixtures of relatively non-swelling solvents such as alkanes with nitrobenzene are preferred.

The surface-functionalized beads described herein are useful, for example, in solid-phase organic synthesis, solid-phase peptide synthesis, and scavenging of reaction byproducts. Typically, coupling reactions between the surface-functionalized beads and reagents in solution occur faster than with conventional functionalized polymer beads. For example, when a 2-chlorotrityl-chloride functional group on a surface-functionalized bead described herein reacts with a given concentration of a protected amino acid reagent in the presence of typical coupling reagents used in peptide synthesis, the reaction typically is complete in the same time or a shorter time than that observed for a conventional functionalized bead, as demonstrated below in Example 8 and Table 4. Coupling efficiency for reaction of the surface functionalized bead of this invention with a protected amino acid residue is greater than that of conventional beads, as demonstrated below by weight gain of the beads in Example 6 and Table 2, and by HPLC measurements of cleaved amino acid in Example 7 and Table 3.

Typical loading of amino acid, with or without typical protecting groups well known in peptide synthesis, onto the surface-functionalized beads of this invention is from 0.2 meq/g to 1.0 meq/g, based on the weight of the unloaded beads. In one embodiment of the invention, preferably, at least 0.25 meq/g is loaded, more preferably at least 0.3 meq/g, more preferably at least 0.5 meq/g, and most preferably at least 0.6 meq/g. Preferably, the loading is no more than 0.9 meq/g, more preferably no more than 0.8 meq/g, and most preferably no more than 0.7 meq/g. In another embodiment of the invention, preferably, at least 0.6 meq/g is loaded, more preferably at least 0.7 meq/g, more preferably at least 0.8 meq/g, and most preferably at least 0.9 meq/g. Preferably, the loading is no more than 1.2 meq/g, more preferably no more than 1.1 meq/g, and most preferably no more than 1.0 meq/g.

**Comparative Example: Internal and Surface Functionalization of Pre-Swelled Crosslinked Polystyrene Beads**

A 1L round bottom flask fitted with an overhead stirrer, $N_2$ inlet fitted with a pressure relief upstream, and a thermocouple was purged with a light positive pressure of nitrogen (sweep against open stopper while making additions). Nitrobenzene (400 mL) was charged and held at room temperature. A polystyrene resin (40 g, 0.379 mol) was charged against the nitrogen sweep and stirred for 1/2 hour. Chlorobenzoyl chloride (24.89 g, 0.142 mol) was charged to the flask and stirred for 15 minutes. Inside a glove bag filled with nitrogen, aluminum chloride (18.96 g, 0.142 mol) was weighed into a sealed bottle, which then was charged into the reaction flask against the nitrogen sweep. The contents of the flask were heated to 30°C and held for 4 hours. The reaction mixture was poured into a buchner filter funnel, and the reaction flask washed with a small amount of nitrobenzene to complete transfer. The filter was drained to resin level, and nitrobenzene (280 mL, 1 bed volume) was added, and the filter drained again. Tetrahydrofuran ("THF") (2 bed volumes) was added on top of resin bed, which was allowed to drain. The color was removed as the THF replaced the nitrobenzene. One bed volume of 4:1 $THF:H_2O$ was added and the resin was re-suspended, then the filter was drained to the resin level and one bed volume of THF was added on top of the resin. The filter was allowed to drain to the resin level. One bed volume of THF was added and the resin was re-suspended, then the filter was drained to the resin level and one bed volume of THF was added on top of the resin. The filter was allowed to drain to the resin level. One bed volume of methanol was added on top of resin. The filter was allowed to drain to the resin level. One bed volume of methanol was added and the resin was re-suspended, then the filter was drained to the resin level and one bed volume of methanol was added on top of the resin. The filter was allowed to drain to the resin level. Minimal vacuum was applied to remove excess solvent. The resin was dried in a 35°C vacuum oven to a constant weight.

**Example: Surface Functionalization of a Crosslinked Polystyrene Bead by Functionalization of Unswelled Beads**

A 1L round bottom flask fitted with an overhead stirrer, $N_2$ inlet fitted with a pressure relief upstream, and a thermocouple was purged with a light positive pressure of nitrogen (sweep against open stopper while making additions). Nitrobenzene (400 mL) was charged and held at room temperature. Inside a glove bag filled with nitrogen, aluminum chloride (18.96 g, 0.142 mol) was weighed into a sealed bottle, which then was charged into the reaction flask against the nitrogen sweep. After the aluminum chloride was dissolved (ca. 5 minutes), chlorobenzoyl chloride (24.89 g, 0.142 mol) was charged to the flask and stirred for 5 minutes. A polystyrene resin (40 g, 0.379 mol) was charged against the nitrogen sweep and stirred for 1/2 hour. The contents of the flask were heated to 30°C and held for an additional 3.5 hours. The reaction mixture was poured into a buchner filter funnel, and the reaction flask washed with a small amount of nitrobenzene to complete transfer. The filter was drained to resin level, and nitrobenzene (280 mL, 1 bed volume) was added, and the filter drained again. Tetrahydrofuran ("THF") (2 bed volumes) was added on top of resin bed, which was allowed to drain. The color was removed as the THF replaced the nitrobenzene. One bed volume of 4:1 $THF:H_2O$ was added and the resin was re-suspended, then the filter was drained to the resin level and one bed volume of THF was added on top of the resin. The filter was allowed to drain to the resin level. One bed volume of THF was added and the resin was re-suspended, then the filter was drained to the resin level and one bed volume of THF was added on top of the resin. The filter was allowed to drain to the resin level. One bed volume of methanol was added on top of the resin. The filter was allowed to drain to the resin level. One bed volume of methanol was added and the resin was re-suspended, then the filter was drained to the resin level and one bed volume of methanol was added on top of the resin. The filter was allowed to drain to the resin level. Minimal vacuum was applied to remove excess solvent. The resin was dried in a 35°C vacuum oven to a constant weight.

**Example: Surface Functionalization of Crosslinked Polystyrene Beads by Selection of Functionalization Solvent**

A 1L round bottom flask fitted with an overhead stirrer, $N_2$ inlet fitted with a pressure relief upstream, and a thermocouple is purged with a light positive pressure of nitrogen (sweep against open stopper while making additions). Nitroethane (400 mL) is charged and held at room temperature. A polystyrene resin (40 g, 0.379 mol) is charged against the nitrogen sweep and stirred for 1/2 hour. Chlorobenzoyl chloride (24.89 g, 0.142 mol) is charged to the flask and stirred for 15 minutes. Inside a glove bag filled with nitrogen, aluminum chloride (18.96 g, 0.142 mol) is weighed into a sealed bottle, which then is charged into the reaction flask against the nitrogen sweep. The contents of the flask are heated to 30°C and held for an additional 3.75 hours. The reaction mixture is poured into a buchner filter funnel, and the reaction flask washed with a small amount of nitrobenzene to complete transfer. The filter is drained to resin level, and nitrobenzene (280 mL, 1 bed volume) is added, and the filter drained again. Tetrahydrofuran ("THF") (2 bed volumes) is added on top of the resin bed, which is allowed to drain. The color is removed as the THF replaces the nitrobenzene. One bed volume of 4:1 THF:$H_2O$ is added and the resin is re-suspended, then the filter is drained to the resin level and one bed volume of THF is added on top of the resin. The filter is allowed to drain to the resin level. One bed volume of THF is added and the resin is re-suspended, then the filter is drained to the resin level and one bed volume of THF is added on top of the resin. The filter is allowed to drain to the resin level. One bed volume of methanol is added on top of resin. The filter is allowed to drain to the resin level. One bed volume of methanol is added and the resin is re-suspended, then the filter is drained to the resin level and one bed volume of methanol is added on top of the resin. The filter is allowed to drain to the resin level. Minimal vacuum is applied to remove excess solvent. The resin is dried in a 35°C vacuum oven to a constant weight.

### Example: Surface Functionalization of Crosslinked Polystyrene Beads by Use of a Mixed Functionalization Solvent

A 1L round bottom flask fitted with an overhead stirrer, $N_2$ inlet fitted with a pressure relief upstream, and a thermocouple is purged with a light positive pressure of nitrogen (sweep against open stopper while making additions). Nitrobenzene (60 mL) and Heptane (440 mL) are charged and held at room temperature. A polystyrene resin (40 g, 0.379 mol) is charged against the nitrogen sweep and stirred for 1/2 hour. Chlorobenzoyl chloride (24.89 g, 0.142 mol) is charged to the flask and stirred for 15 minutes. Inside a glove bag filled with nitrogen, aluminum chloride (18.96 g, 0.142 mol) is weighed into a sealed bottle, which then is charged into the reaction flask against the nitrogen sweep. The contents of the flask are heated to 30°C and held for 4 hours. The reaction mixture is poured into a buchner filter funnel, and the reaction flask washed with a small amount of nitrobenzene to complete transfer. The filter is drained to resin level, and nitrobenzene (280 mL, 1 bed volume) is added, and the filter drained again. Tetrahydrofuran ("THF") (2 bed volumes) is added on top of resin bed, which is allowed to drain. The color is removed as the THF replaces the nitrobenzene. One bed volume of 4:1 THF:$H_2O$ is added and the resin is re-suspended, then the filter is drained to the resin level and one bed volume of THF is added on top of the resin. The filter is allowed to drain to the resin level. One bed volume of THF is added and the resin is re-suspended, then the filter is drained to the resin level and one bed volume of THF is added on top of the resin. The filter is allowed to drain to the resin level. One bed volume of methanol is added on top of resin. The filter is allowed to drain to the resin level. One bed volume of methanol is added and the resin is re-suspended, then the filter is drained to the resin level and one bed volume of methanol is added on top of the resin. The filter is allowed to drain to the resin level. Minimal vacuum is applied to remove excess solvent. The resin is dried in a 35°C vacuum oven to a constant weight.

### Example: General Procedure for Final Functionalization of Crosslinked Beads

In an oven dried four neck round bottom flask (equipped with a stirrer, a condenser w/nitrogen bubbler, a temperature controller, and a septum) was taken the THF and the dried bead resulting from any of the previous Examples (10:1, volume:weight). The mixture was stirred for 15 minutes. Phenyl lithium (1.25 equivalents) was added drop wise over 10 minutes. The temperature was kept <30°C by an ice/water bath. The reaction mixture was then stirred at ambient temperature for 1 hour. Quenching was accomplished by drop wise addition of 10% aqueous HCl, keeping the reaction temperature below 30°C. The mixture was stirred for 1 hour. The contents are then transferred to a sinter glass funnel and drained to bed height. The resin was then re-suspended in 1 bed volume of 4:1 THF/ 10%HCl(v/v) and allowed to drain to bed height slowly. The resin was re-suspended with 1 bed volume of 4:1 THF/ water and allowed to drain. The bed was then re-suspended and drained 3 times with 1 bed volume of THF, followed by re-suspending/draining 3 times with 1 bed volume of methanol. A final rinse through of the bed is done with 1 bed volume of methanol. Vacuum was applied to remove excess solvent and then the beads were dried in a 35°C vacuum oven.

In an oven dried four neck round bottom flask (equipped with a stirrer, a temperature controller, a condenser w/nitrogen bubbler, and a stopper) was added the methylene chloride and the dried bead from the previous step (10:1). Added thionyl chloride (5 equivalents) drop-wise followed by N,N-dimethylformamide (5 mole % based on thionyl chloride). The mixture was warmed to reflux (37°C) for 4 hours. After cooling to ambient temperature, the reaction mixture was transferred to a nitrogen purged sintered glass funnel and drained to bed height. The bed was then re-suspended and drained twice with 1 bed volume of methylene chloride. It was then further washed by re-suspending/draining three times with 1 bed volume of anhydrous hexane. Purged through the bed with nitrogen to remove excess solvent and then placed the beads in a vacuum oven at ambient temperature. The trityl chloride functionalized bead resulting from this preparation is useful, for example, in solid phase peptide synthesis.

## Example : Swelling of Crosslinked Polystyrene Beads in Various Solvents

Crosslinked polystyrene beads made using 1% and approximately 1.5% divinylbenzene as a crosslinker, and having a volume when dry of 1.65 mL/g were swelled in solvents, with the results presented below in Table 1 in mL/g. Solvent ratios are volume:volume.

Table1

| Solvent | 1.5% crosslinker | 1% crosslinker |
|---|---|---|
| nitromethane | 2.5 | N/A |
| nitropropane | 3.7 | 4.05 |
| 1:1, nitropropane:heptane | 3.6 | 4.3 |
| 1:2, nitropropane:heptane | 3.5 | 3.7 |
| 1:3, nitropropane:heptane | 3.3 | 3.55 |
| nitrobenzene | 4.0 | 5.3 |
| 1:1, nitrobenzene:heptane | 4.6 | 5.6 |
| 1:2, nitrobenzene:heptane | 4.5 | 5.05 |
| 1:3, nitrobenzene:heptane | 4.2 | 4.3 |
| methanol | 1.7 | N/A |
| heptane | 1.9 | N/A |

## Example: Loading of Surface-Functionalized Crosslinked Beads with Fmoc-L-Leucine

A 2-chlorotrityl chloride resin produced according to Example 4 was loaded with Fmoc-L-Leucine, treated with methanol to remove residual reactive chloride and dried. The weight gain was used to quantify loading. The resin was assumed to have a capacity of 1.3 mmol/g. The relatively minor molecular weight effect of the methoxy end-capping was ignored. The resin was cleaved with 1 % TFA/DCM, and the solution was analyzed by HPLC to determine the cleaved yield (recovery) of amino acid.

Each sample of the resin (1.0000 +/- 0.05 g) was weighed into a 60 mL glass synthesizer vessel with a side port and a removable disk. The resin in the synthesizer was pre-swelled with dichloromethane (DCM). The DCM was drained and to each synthesizer was added a solution of Fmoc-L-Leu-OH and diisopropylethylamine (DIEA) in 10 ml DCM. Slow nitrogen agitation was started. For the five resins of this invention, the quantities, in grams, of Fmoc-L-Leu-OH were (3.181, 0.597, 0.358, 0.299, 0.239) and of DIEA, in mL, were (1.568, 0.294, 0.177, 0.147, 0.118) per sample, respectively. Each mixture was allowed to react at ambient temperature for two hours, then the solution was drained and any remaining trityl chloride end groups were capped by treatment for at least 30 minutes with DIEA (1 mL) in methanol (9 mL). Each sample of resin was washed with 5 x 10 mL portions of DCM and transferred to a tared 30 mL fritted glass funnel, then washed with another 2 x 10 mL portions of DCM. Each loaded resin was then de-swelled with 4 x 10mL portions of isopropanol (IPA) and partially dried by pulling air through the filter cake with vacuum, then drying the filter and resin overnight in a vacuum oven at 30°C. The filter and resin were then re-weighed and the difference in mass calculated. Mass of Leu = Final wt-(filter tare+1.000g resin). Loading efficiency = (weight of Leu on resin/weight of Leu charged)* 100. The weight gain and loading efficiency are reported in Table 2 for five resins of this invention (RH1-RH5) and for three competitive resins processed according to the procedures given in this Example (CM1-CM3). The cleaved yield for the same resins is reported in Table 3. The amount of amino acid (AA) is in mmol, the weight gain (gain) in mmol, and the loading efficiency (eff.) in %.

The five resins of the present invention have higher loading efficiency than any of the competitive resins in Table 2. This higher load efficiency is in the range of an about 7.5 to about 28 % improvement over the conventional resins in Table 2.

Table 2:

| Weight Gain and Loading Efficiency Comparison | | | | | | | |
|---|---|---|---|---|---|---|---|
| amount of AA | 0.61 | 0.68 | 0.73 | 0.84-0.85 | 0.97 | 1.01 | 1.05 | 1.26 |

Table 2: (continued)

| Weight Gain and Loading Efficiency Comparison | | | | | | | |
|---|---|---|---|---|---|---|---|
| gain, RH1 | | 0.54 | | 0.81 | | 0.97 | | |
| gain, RH2 | | 0.31 | | 0.52 | | 0.79 | | |
| gain, RH3 | | 0.49 | | 0.60 | | 0.82 | | |
| gain, RH4 | | 0.66 | | 0.75 | | 0.98 | | |
| gain, RH5 | | 0.63 | | 0.82 | | 0.86 | | |
| avg. gain, RH1-RH5 | | 0.53 | | 0.70 | | 0.88 | | |
| gain, CM1 | | 0.34 | | 0.53 | | 0.81 | | |
| gain, CM2 | 0.26 | | 0.32 | | 0.73 | | | |
| gain, CM3 | | | | 0.46 | | | 0.80 | 0.88 |
| eff., RH1 | | 80.5 | | 95.8 | | 96.1 | | |
| eff., RH2 | | 45.5 | | 61.4 | | 77.7 | | |
| eff., RH3 | | 72.5 | | 70.9 | | 80.8 | | |
| eff., RH4 | | 97.7 | | 88.5 | | 97.1 | | |
| eff., RH5 | | 93.2 | | 96.8 | | 84.9 | | |
| avg. eff., RH1-RH5 | | 77.9 | | 82.7 | | 87.3 | | |
| eff., CM1 | | 49.9 | | 62.4 | | 79.8 | | |
| eff., CM2 | 42.5 | | 44.4 | | 75.2 | | | |
| eff., CM3 | | | | 55.2 | | | 76.4 | 70.0 |
| CM2 is a resin available from Novabiochem under the name 2-Chlorotritylchloride Resin (100-200 mesh), 1 % DVB; Cat. No. 01-64-0114 | | | | | | | | |
| CM3 is a resin available from Polymer Labs under the name Cl-Trt-Cl Resin (75-150 micron), 1% DVB, Part No. 3473-2799 | | | | | | | | |

### Example: Cleavage of Fmoc-L-Leucine from Surface-Functionalized Beads

Each sample of the resin (1.0000 +/- 0.05 g) was weighed into a fritted glass filter. The resin was pre-swelled by agitating the funnel with DCM (10 mL), the DCM was drained, and the resin washed 3 x 10 mL DCM. The resin bound Fmoc-L-Leu-OH was cleaved by agitating with 9x 10 ml of 1% TFA/DCM (v:v), draining into a 100 mL volumetric flask, and filling to the mark with DCM. The contents of the flask were agitated to provide the sample solution to be analyzed by HPLC.

The sample solution was injected into a liquid chromatographic system capable of generating a binary solvent gradient, and equipped with a sample injector, a variable wavelength detector and electronic data acquisition system (HP 1090 with ChemStation™ software). Column: YMC ODS-AQ, S-3 , 120 A, 50 mm X 4 mm ID column Catalog # AQ12S030504WT

| Conditions: | |
|---|---|
| Flow rate | 1.5 mls/min |
| Program | 40% B, hold 10.0 min 40 % B to 90% B over 2.5 minutes, hold 1 minute 90% B over 2 minutes hold 10 minutes until next injection. |
| Injection vol. | 10uL |

Detection: photodiode array detector 265 nm bandwidth 16 nm, ref: 350 nm, bw 100 nm, or variable wavelength UV Detector at 265 nm.

Standard preparation:

Approximately 5.0 mg of the Fmoc-L-Leu-OH reference standard were weighed into a 25 mL volumetric flask. The standard was dissolved in about 10 mL of acetonitrile (often requires sonication). Water (12 mL) was added, the contents were mixed, and the flask was allowed to come to ambient temperature. The flask was filled to the mark with water and the contents were mixed.

Sample preparation:

The sample solution (5.00 mL) was measured into a 25 mL volumetric flask, and reduced to dryness at ambient temperature with a gentle nitrogen stream. The residue was dissolved in 10 mL of acetonitrile (often requires sonication). Water (12 mL) was added, the contents were mixed well and the flask allowed to come to ambient temperature. The flask was filled to the mark with water and agitated.
A blank (water: acetonitrile 3:2) was injected and the gradient program started. Concomitantly, the sample and standard were injected.
The loading of the Fmoc-L-Leu on the resin was calculated by:

Fmoc-L-Leu in sample flask = Area sample/Area standard X Wt of standard x Purity of standard/ 25.0 mL X 25.0 mL/ 5.0 mL

Resin Loading (mmol/g of dry resin) = Amt of Fmoc L-Leu-OH in sample flask/ Wt of

loaded resin g X 353.4

[353.4 is the molecular weight of the Fmoc-L-Leu-OH]
Results for the amount of cleaved Fmoc L-Leu-OH in mmol ("AA") for each amount of amino acid used to load the resins initially for the RH to RH5 and CM materials are reported in Table 3. Load efficiencies are also reported, assuming that the cleaved amount equals the amount bound to the resin.

Table 3:

| Cleaved Amino Acid Yield Comparison | | | |
|---|---|---|---|
| amount of AA | 0.68 | 0.85 | 1.01 |
| AA, RH1 | 0.56 | 0.85 | 0.99 |
| AA, RH2 | 0.31 | 0.51 | 0.82 |
| AA, RH3 | 0.50 | 0.61 | 0.84 |
| AA, RH4 | 0.63 | 0.72 | 0.99 |
| AA, RH5 | 0.64 | 0.87 | 0.88 |
| avg. AA, RH1-RH5 | 0.53 | 0.71 | 0.90 |
| AA, CM1 | 0.33 | 0.60 | 0.85 |
| eff., RH1 | 82.4 | 100.0 | 98.0 |
| eff., RH2 | 45.6 | 60.0 | 81.2 |
| eff., RH3 | 73.5 | 71.8 | 83.2 |
| eff., RH4 | 92.6 | 84.7 | 98.0 |
| eff., RH5 | 94.1 | 102.4 | 87.1 |
| avg. eff., RH1-RH5 | 77.6 | 83.8 | 89.5 |
| eff., CM 1 | 48.5 | 70.6 | 84.2 |

The cleaved amino acid yield for the resins of the present invention was 5-20% greater than the conventional resins. It is appreciated that the cleaved peptide fragment yield will be higher than when the same fragments are cleaved from the competitive resin.

### Example: Peptide Build Kinetic Efficiency Comparison

This Example describes the preparation of a nine-peptide fragment of the peptide known as T-20, described in U.S. Pat. No. 6,015,881, Table 1, as Peptide No. 11, containing amino acids 17-26. The kinetics of the reaction are followed by sampling resin periodically during the coupling and running a Kaiser test to determine the presence of any unreacted primary amine. The resin according to Example 4 is compared with two competitive resins, one from Novabiochem, and the other from Polymer Labs.

A 2-chlorotrityl chloride resin produced according to Example 4 was loaded with Fmoc-L-Leucine, treated with methanol to remove residual reactive chloride and dried. A sample of the resin (1.0 g) was weighed into a 60 mL glass synthesizer vessel with a side port and a removable disk. DCM (10 mL) was charged to the vessel and agitated with nitrogen for 30 minutes, then drained. The leucine derivatized resin is then deprotected by charging 10 mL of a 25% solution of piperidine in N-methylpyrrolidone (NMP), agitating for 10 minutes, draining and repeating once. The deprotection residue was removed by washing with 7x 10 mL volumes of NMP. The activated ester of next amino acid in sequence was prepared by dissolving 1.5 eq of amino acid (Fmoc-glu(t-Bu)-OH was the first added in this sequence, see table for charges and formula weights), 1.5 eq of 1-hydroxybenzotriazole (HOBT) (0.149 g) and 1.5 eq of DIEA (0.126 g) into 7.5 mL of NMP at room temperature. The solution was then chilled and 1.5 eq of O-benzotriazol-1-yl-N, N,N',N',-tetramethyluronium hexafluorophosphate (HBTU) (0.370 g) was added and stirred for 30 minutes. DCM (2.5 mL) was then charged to the solution and allowed to stand for 30 minutes. The activated amino acid solution was then charged to the drained resin and agitated with nitrogen. Samples were obtained and analyzed (Kaiser test) each 15 minutes and the results recorded. Upon completion of the reaction the resin was drained and washed with NMP (3x10 mL). This process is then repeated from the deprotection with piperidine for the rest of the amino acids in the sequence. (Glu(tBu), Lys(Boc), Asn(trt), Glu(tBu), Gln(trt), Glu(tBu), leu, leu, ).

The results for the three resins are presented below in Table 4, with times expressed as the time to a negative Kaiser Test.

Table 4:

| Peptide Synthesis Efficiency Comparison | | | |
|---|---|---|---|
| Amino Acid | R+H | Polymer Labs | Nova Biochem |
| 1 | 45 | 60 | 60 |
| 2 | 30 | 60 | 60 |
| 3 | 60 | 60 | 60 |
| 4 | 30 | 45 | 45 |
| 5 | 60 | 60 | 60 |
| 6 | 30 | 45 | 45 |
| 7 | 30 | 45 | 30 |
| 8 | 30 | 45 | 30 |
| 9 | 30 | 45 | 45 |
| Total Cycle Time | 345 | 465 | 435 |

The time required for complete reaction with each amino acid added to the growing chain on the bead of this invention is the same or less than for the conventional beads.

Appendix 1 AA usage

| Monomer | fwt | g required |
|---|---|---|
| FMOC Glu (t-Bu) | 425.48 | 0.415 |
| FMOC Lys (Boc) | 468.55 | 0.457 |
| FMOC Asn (trt) | 596.68 | 0.582 |
| FMOC Glu (t-Bu) | 425.48 | 0.415 |
| FMOC Gln (trt) | 610.71 | 0.595 |
| FMOC Glu (t-Bu) | 425.48 | 0.415 |
| FMOC Leu | 353.42 | 0.345 |
| FMOC Leu | 353.42 | 0.345 |
| FMOC Glu (t-Bu) | 425.48 | 0.415 |
| HOBT | 153.15 | 0.149 |
| DIEA | 129.25 | 0.126 |
| HBTU | 379.25 | 0.370 |

| | | | | |
|---|---|---|---|---|
| LeuCT-resin (g)= | 1.00 | Total NMP | 1035.5mL |
| Loading Level (mmol/g)= | 0.65 | Total DCM | 72.5mL |
| Number of samples= | 1.00 | Total Piperdine | 32mL |
| Total resin(g)= | 1.00 | Total HOBT | 1.344g |
| Total mmol= | 0.65 | Total DIEA | 1.134g |
| Eq. of Monomer Charge= | 1.50 | Total HBTU | 3.328g |
| Coupling cycles per step= | 1.00 | | |
| Monomer usage/Cycle (mmol)= | 0.975 | | |

| | |
|---|---|
| AA Added | 9 |

Appendix 2 Solvent usage

|  | SetUp | each sample each cycle | Total each cycle | Total complete |
|---|---|---|---|---|
| Resin | 1.00g | 1.00g | 1.00g |
| | | | | |
| Swell | | | | |
| DCM | 10mL | 10 | 10 |
| Stir for 15 min | | | | |
| | | | | |
| Deprotect | | | | |
| 20%Piperidine in NMP | 10mL | 10 | |
| Cycles | 2 | | |
| Total | 20mL | 20 | 160 |
| Stir for 10 min/cycle | | | | |
| | | | | |
| Wash 1 | | | | |
| NMP | 10mL | 10 | |
| Cycles | 7 | | |
| Total | 70mL | 70 | 560 |
| | | | | |
| Coupling | | | | |
| MonomersSee AA Sheet | | | |
| NMP | 7.5mL | 7.5 | 67.5 |
| DCM | 2.5mL | 2.5 | 22.5 |
| | | | | |
| Wash 2 | | | | |
| NMP | 10 | 10 | |
| Cycles | 3 | | |
| Total | 30 | 30 | 240 |

Final Wash

| | | | |
|---|---|---|---|
| NMP | 10 | 10 | |
| Cycles | 4 | | |
| Total NMP | 40 | 40 | 40 |
| DCM | 10 | 10 | |
| Cycles | 4 | | |
| Total DCM | 40 | 40 | 40 |

| | |
|---|---|
| Total NMP | 1035.5 mL |
| Total DCM | 72.5 mL |
| Total Piperdine | 32 mL |

It is appreciated that improved accessibility leads to more efficient coupling and washing resulting in decreased solvent usage at commercial scale. By way of example a 10% reduction in NMP usage will provide a 15500 L reduction per 100 kg of peptide product.

Appendix 3 Kaiser Test Method

The Kaiser test is a test for primary amines and is employed to determine the extent of peptide coupling. A sample of loaded resin (3-20 mg) is placed into a culture tube and evaporated to dryness. The resin is then washed 3 times with ethanol. Five drops of reagent 1 (KCN in pyridine) is added along with 3 drops of reagent 2 (ninhydrin solution) and 3 drops of reagent 3 (phenol in Ethanol). The solution is diluted to 0.5 mL then heated to 75°C for 10 minutes. After 10 minutes the tubes are chilled in a cold water bath. The beads are then observed in front of a white background. A negative test is indicated if the solution is yellow and the beads are transparent. A blue or violet color indicates the presence of free amines and incomplete coupling.

In yet another variant, the invention provides an improved process for making a T-20 or a T-1249 composition, or a fragment of a T-20 or a T-1249 composition using a low void space resin optionally loaded with. an amino acid or amino acid derivative to create one or more T-20 or T-1249 fragments.

The present invention uses, by way of non-limiting example, a crosslinked polymeric bead comprising a polymer having from 0.5 mole percent to 2 mole percent crosslinker; wherein the bead has a diameter no greater than 200 μm, no void spaces having a diameter greater than about 5 μm, and less than 5 weight percent of organic extractables.

The resins of the present invention can be prepared by the following exemplary method. The method includes the steps of: (a) preparing a suspension polymerization mixture in a vessel; the mixture comprising: (i) a monomer mixture comprising at least one vinyl monomer and at least one crosslinker; and (ii) from 0.25 mole percent to 1.5 mole percent of at least one free radical initiator; (b) removing oxygen from the vessel by introducing an inert gas for a time sufficient to produce an atmosphere in the vessel containing no more than 5 percent oxygen; (c) allowing the monomer mixture to polymerize; and (d) washing the bead with an aprotic organic solvent. Of course, it is appreciated that other methods can also be used to obtain the resins used in the present invention having the qualities of being low void space resins.

As used herein the term "(meth)acrylic" refers to acrylic or methacrylic. The term "vinyl monomer" refers to a monomer suitable for addition polymerization and containing a single polymerizable carbon-carbon double bond. The term "styrene polymer" indicates a copolymer polymerized from a vinyl monomer or mixture of vinyl monomers containing at least 50 weight percent, based on the total monomer weight, of styrene monomer, along with at least one crosslinker. Preferably a styrene polymer is made from a mixture of monomers that is at least 75% styrene, more preferably at least 90% styrene, and most preferably from a mixture of monomers that consists essentially of styrene and at least one vinylaromatic crosslinker. The lightly crosslinked polymeric bead of this invention contains monomer residues from at least one monomer having one copolymerizable carbon-carbon double bond and at least one crosslinker. The monomer residues derived from the crosslinker are from 0.5 mole percent to 2 mole percent based on the total of all monomer reisdues.

The term "organic extractables" refers to low-molecular weight organic substances that are removed from the polymeric bead by washing in an organic solvent which causes the bead to swell. Examples of organic extractables include, for example, unreacted monomer or crosslinker, or low molecular weight oligomers. Preferably, the amount of organic extractables is measured by suspending the beads in tetrahydrofuran or dichloromethane for two hours, rinsing in a column with five volumes of solvent/volume of beads, drying and determining the weight loss of the beads.

Preferably, organic extractables are removed from the beads of the present invention by treatment with a non-protic organic solvent, preferably one that is not an aliphatic hydrocarbon, for example, halogenated hydrocarbons, cyclic ethers, ketones and aromatic hydrocarbons. Particularly preferred solvents are dichloromethane, dichloroethane, chloroform, chlorobenzene, o-dichlorobenzene, tetrahydrofuran, dioxane, acetonitrile, acetone, xylene and toluene. Preferably, the beads of the present invention contain less than 4 weight percent of organic extractables, more preferably less than 3 weight percent, more preferably less than 2 weight percent, more preferably less than 1 weight percent, and most preferably the beads are substantially free of organic extractables. In one embodiment of the invention, the beads contain less than 3 weight percent of unreacted monomer, more preferably less than 2 weight percent, more preferably less than 1 weight percent, and most preferably the beads are substantially free of unreacted monomer. Typically, the beads contain low levels of extractables and unreacted monomer even prior to washing with an aprotic organic solvent. When the polymer is a styrene polymer crosslinked with divinylbenzene ("DVB"), unreacted monomer may comprise unpolymerized ethylvinylbenzene ("EVB"), a common impurity in commercial divinylbenzene, and possibly also unreacted styrene. Commercial divinylbenzene typically has a purity from 55% to 80%, with the remainder largely consisting of ethylvinylbenzene. Preferably, divinylbenzene with a purity of at least 60% is used, more preferably at least 70%, more preferably at least 75%, and most preferably at least 80%.

A polymeric bead used in the present invention generally is a spherical copolymer bead having a particle diameter no greater than 200 microns ($\mu$m), preferably no greater than 170 $\mu$m, more preferably no greater than 150 $\mu$m, more preferably no greater than 125 $\mu$m, and most preferably no greater than 100 $\mu$m. Preferably, the bead has no void spaces having a diameter greater than 3 $\mu$m, more preferably no void spaces having a diameter greater than 2 $\mu$m, and most preferably no void spaces having a diameter greater than 1 $\mu$m. Typically, void spaces are readily apparent upon surface examination of the bead by a technique such as light microscopy.

The polymeric bead used in the present invention preferably is produced by a suspension polymerization. A typical bead preparation, for example, may include preparation of a continuous aqueous phase solution containing typical suspension aids, for example, dispersants, protective colloids and buffers. Preferably, to aid in production of the relatively small beads of the present invention, a surfactant is included in the aqueous solution, preferably a sodium alkyl sulfate surfactant, and vigorous agitation is maintained during the polymerization process. The aqueous solution is combined with a monomer mixture containing at least one vinyl monomer, at least one crosslinker and at least one free-radical initiator. Preferably, the total initiator level is from 0.25 mole percent to 1.5 mole %, based on the total monomer charge, preferably from 0.4 mole percent to 1 mole percent, more preferably from 0.4 mole percent to 0.8 mole percent, and most preferably from 0.5 mole percent to 0.7 mole percent. The mixture is purged of most of the oxygen by introducing an inert gas until the oxygen level in the atmosphere in the reaction vessel (head space) is less than 5%, preferably less than 3%, more preferably less than 2%, and most preferably less than 1 %. Preferably, the inert gas is introduced into the aqueous solution and the monomer mixture, as well as the head space. The mixture of monomers is then polymerized at elevated temperature. Preferably, the polymerization is continued for a time sufficient to reduce the unreacted vinyl monomer content to less than 1 % of the starting amount. The resulting bead is then isolated by conventional means, such as dewatering, washing with an aprotic organic solvent, and drying.

Where one or more of the monomers contains a phenolic polymerization inhibitor, the aqueous phase of the suspension polymerization mixture is maintained at a pH from 9 to 11.5 to extract the phenolic inhibitor from the monomer phase as much as possible. Preferably, the pH of the aqueous phase is from 9.5 to 11.

Crosslinkers are monomers having 2 or more copolymerizable carbon-carbon double bonds per molecule, such as: divinylbenzene, divinyltoluene, divinylxylene, trivinylbenzene, trivinylcyclohexane, divinylnaphthalene, trivinylnaphthalene, diethyleneglycol divinylether, ethyleneglycol dimethacrylate, polyethyleneglycol dimethacrylate triethyleneglycol dimethacrylate, trimethylolpropane trimethacrylate, allyl methacrylate, 1,5-hexadiene, 1,7-octadiene or 1,4-bis(4-vinylphenoxy)butane; it is understood that any of the various positional isomers of each of the aforementioned crosslinkers is suitable. Preferred crosslinkers are divinylbenzene, divinyltoluene, trivinylbenzene or 1,4-bis(4-vinylphenoxy)butane. The most preferred crosslinker is divinylbenzene.

Suitable monounsaturated vinylaromatic monomers that may be used in the preparation of the bead used in the present invention include, for example, styrene, $\alpha$-methylstyrene, $(C_1$-$C_4)$alkyl-substituted styrenes and vinylnaphthalene; preferably one or more monounsaturated vinylaromatic monomer is selected from the group consisting of styrene and $(C_1$-$C_4)$alkyl-substituted styrenes. Included among the suitable $(C_1$-$C_4)$alkyl-substituted styrenes are, for example, ethylvinylbenzenes, vinyltoluenes, diethylstyrenes, ethylmethylstyrenes, dimethylstyrenes and isomers of vinylbenzyl chloride; it is understood that any of the various positional isomers of each of the aforementioned vinylaromatic monomers is suitable.

Optionally, non-aromatic vinyl monomers, such as aliphatic unsaturated monomers, for example, acrylonitrile, glycidyl methacrylate, (meth)acrylic acids and amides or $C_1$-$C_6$ alkyl esters of (meth)acrylic acids may also be used in addition to the vinylaromatic monomer. When used, the non-aromatic vinyl monomers typically comprise as polymerized units, from zero to 20%, preferably from zero to 10%, and more preferably from zero to 5% of the copolymer, based on the total monomer weight used to form the copolymer.

Preferred vinyl monomers are the vinylaromatic monomers; more preferably styrene, isomers of vinylbenzyl chloride, and $\alpha$-methylstyrene. The most preferred vinyl monomer is styrene.

Polymerization initiators useful preparing the beads used in the present invention include monomer-soluble initiators such as peroxides, hydroperoxides, peroxyesters and related initiators; for example benzoyl peroxide, *tert*-butyl hydroperoxide, cumene peroxide, tetralin peroxide, acetyl peroxide, caproyl peroxide, *tert*-butyl peroctoate (also known as *tert*-butylperoxy-2-ethylhexanoate), tert-amyl peroctoate, *tert*-butyl perbenzoate, *tert*-butyl diperphthalate, dicyclohexyl peroxydicarbonate, di(4-*tert*-butylcyclohexyl)peroxydicarbonate and methyl ethyl ketone peroxide. Also useful are azo initiators such as azodiisobutyronitrile, azodiisobutyramide, 2,2'-azo-bis(2,4-dimethylvaleronitrile), azo-*bis*($\alpha$-methyl-butyronitrile) and dimethyl-, diethyl- or dibutyl azo-bis(methylvalerate). Preferred peroxide initiators are diacyl peroxides, such as benzoyl peroxide, and peroxyesters, such as tert-butyl peroctoate and tert-butyl perbenzoate.

Dispersants and suspending agents useful in the present invention are nonionic surfactants having a hydroxyalkylcellulose backbone, a hydrophobic alkyl side chain containing from 1 to 24 carbon atoms, and an average of from 1 to 8, preferably from 1 to 5, ethylene oxide groups substituting each repeating unit of the hydroxyalkyl-cellulose backbone, the alkyl side chains being present at a level of 0.1 to 10 alkyl groups per 100 repeating units in the hydroxyalkylcellulose backbone. The alkyl group in the hydroxyalkylcellulose may contain from 1 to 24 carbons, and may be linear, branched or cyclic. More preferred is a hydroxyethylcellulose containing from 0.1 to 10 $(C_{16})$alkyl side chains per 100 anhydroglucose units and from about 2.5 to 4 ethylene oxide groups substituting each anhydroglucose unit. Typical use levels of dispersants are from about 0.01 to about 4%, based upon the total aqueous-phase weight.

Optionally, the preparation of the beads may include an enzyme treatment to cleanse the polymer surface of residues of dispersants and suspending agents used during the polymerization. The enzyme treatment typically involves contacting the polymeric phase with the enzymatic material (selected from one or more of cellulose-decomposing enzyme and proteolytic enzyme) during polymerization, following polymerization or after isolation of the polymer. Japanese Patent Applications No. 61-141704 and No. 57-98504 may be consulted for further general and specific details on the use of enzymes during the preparation of polymer resins. Suitable enzymes include, for example, cellulose-decomposing enzymes, such as $\beta$-1,4-glucan-4-glucano-hydrase, $\beta$-1,4-glucan-4-glucanhydrolase, $\beta$-1,4-glucan-4-glucohydrase and? $\beta$-1,4- glucan-4-cellobiohydrase, for cellulose-based dispersant systems; and proteolytic enzymes, such as urokinase, elastase and enterokinase, for gelatin-based dispersant systems. Typically, the amount of enzyme used relative to the polymer is from 2 to 35%, preferably from 5 to 25% and more preferably from 10 to 20%, based on total weight of polymer.

In a preferred embodiment, the beads used in the present invention are lightly crosslinked polymeric bead having no void spaces having a diameter greater than 5 $\mu$m; the beads are produced by a method comprising steps of: (a) preparing a suspension polymerization mixture in a vessel; said mixture comprising: (i) a monomer mixture comprising at least one vinyl monomer and at least one crosslinker; and (ii) from 0.25 mole percent to 1.5 mole percent of at least one free radical initiator; (b) removing oxygen from the suspension polymerization mixture and the vessel by introducing an inert gas for a time sufficient to produce an atmosphere in the vessel containing no more than 5 percent oxygen; (c) allowing the monomer mixture to polymerize; and (d) washing the bead with an aprotic organic solvent. Preferably, the bead made according to this process has no void spaces with a diameter greater than 4 $\mu$m, more preferably no void spaces with a diameter greater than 3 $\mu$m, and most preferably no void spaces with a diameter greater than 1 $\mu$m. Preferably, the bead has less than 5% of organic extractables, more preferably less than 3%, more preferably less than 2%, and most preferably less than 1%. Preferably, the bead has less than 4% of residual monomer, more preferably less than 3%, more preferably less than 2%, and most preferably less than 1%.

Without wishing to be bound by theory, it is believed that the process of this invention facilitates more complete polymerization than previously known processes, and thus reduces the amount of organic extractable materials present in the bead, and therefore also reduces the formation of void spaces in the beads after washing with aprotic organic solvents.

**RESIN PREPARATION EXAMPLE**

Deionized ("DI") water was charged to a round bottom flask, stirred at 150 rpm and heated to 80°C under a nitrogen sweep. When the temperature was reached, the flask was charged slowly with 4.40g of QP-300 (hydroxyethylcellulose dispersant, obtained from Union Carbide Co., Institute, WV). The temperature was maintained for 60 minutes at 80°C, after which the aqueous solution was cooled to 25°C to 30° C. The following were charged to the flask: a solution of 200g DI water and 0.95 g of Marasperse N-22 (sodium lignosulfate dispersant, obtained from Borregaard LignoTech, Rothschild, WI), 2.4 g 50% NaOH, 2.5g boric acid, 0.036g sodium lauryl sulfate and 0.1 g sodium nitrite. The contents of the flask were stirred for 30 minutes. In another example Culminal MHEC 8000 (hydroxyethylcellulose dispersant, obtained from Hercules, Wilmington, DE) can be substituted for the QP-300, Marasperse N-22 and salt.

The monomer mixture was prepared in a separate beaker by charging the following: 6.55g 80% DVB (divinylbenzene), 440.0g styrene, 5.8g Trigonox 21 (t-butyl peroxy-2-ethylhexanoate, obtained from Noury Chemical Corp., Burt, NY). The mixture was transferred to an addition funnel and sparged with nitrogen for 40 minutes.

The agitator speed was adjusted to 275rpm in the round bottom flask containing the aqueous phase before charging the monomer mixture to the flask. The agitator was stopped and the monomer mixture was charged to the aqueous solution, taking care to position the addition funnel so as not to introduce air to the monomer solution. After charging the monomer mixture, agitation was resumed and continued for 30 minutes at 25° C. The temperature was increased to 84° C over 1 hour and maintained there for 12 hours.

The batch was cooled to 45°C, and the pH adjusted to 5.0 with HCl (37%). Cellulase 4000 (19.05g) (cellulase enzyme, obtained from Valley Research, South Bend, IN) was charged to the batch, and stirred for 2 hours at 45°C. After the 2 hour hold a second charge of Cellulase 4000 was added and the temperature maintained for 2 hours at 45°C. At the end of the hold period the batch was cooled to room temperature, removed from the flask and washed with DI water.

Typically, the yield of polymeric beads is approximately 90%, with some polymer lost due to agitator fouling or dispersion in the aqueous phase. The level of residual monomer varies with several parameters, including the thoroughness of the inertion with nitrogen, purity of DVB, and initiator level, as illustrated in the Table. Inertion of reactants or reaction vessel was not performed, except as noted.

Table

| Initiator[1], weight % | DVB purity, % | Residual Styrene, % | Comments |
|---|---|---|---|
| 1.29 | 80 | 3.6 | monomer, aqueous not inerted |
| 1.29 | 80 | 0.6 | full inertion as described in procedure given above |
| 1.29 | 55 | 8.6 | added to achieve same DVB level |
| 1.29 | 80 | 3.7 | |
| 1.29 | 55 | 2.5 | full inertion |
| 1.29 | 80 | 3.6 | |
| 2.30 | 80 | 8.5 | |

1. t-butyl peroxy-2-ethylhexanoate.

Typically, the polymer is washed according to the following procedure. A 4.4 cm diameter, 50 cm long column is loaded with 100 mL of the copolymer. The copolymer is washed with 8 bed volumes of aprotic organic solvent at a flow rate of 0.5 bed volumes/hour in a down flow direction. The bed is washed with 4 bed volumes of methanol or water at a flow rate of 0.5 bed volumes/hour in a down flow direction. The bed is dried in a stream of nitrogen and then dried under vacuum at 45°C for 18 hours.

In one variant of the invention, the resin used comprises a crosslinked polymeric bead having a polymer having from 0.5 mole percent to 2 mole percent crosslinker. The bead has a diameter no greater than 200 μm, no void spaces having a diameter greater than 5 μm, and less than 5 weight percent of organic extractables. In another variant, the polymer has from 0.5% to 1.6% crosslinker and the bead has a diameter no greater than 170 μm. The polymer is a styrene polymer with a divinylbenzene crosslinker in one variant of the invention. The polymer can have from 0.7 mole percent to 1.2 mole percent crosslinker, and the bead may have no void spaces having a diameter greater than 3 μm, and less than 3 weight percent of organic extractables. By way of example, the crosslinked polymeric bead has a diameter no greater than 150 μm.

Another example of creating the resin used in the present uses the following method: (a) preparing a suspension polymerization mixture in a vessel. The mixture comprises: (i) a monomer mixture comprising at least one vinyl monomer and at least one crosslinker; and (ii) from 0.25 mole percent to 1.5 mole percent of at least one free radical initiator; The method next includes removing oxygen from the suspension polymerization mixture and the vessel by introducing an inert gas for a time sufficient to produce an atmosphere in the vessel containing no more than 5 percent oxygen; allowing the monomer mixture to polymerize; and washing the bead with an aprotic organic solvent. The monomer mixture optionallycontains from 0.5 mole percent to 2 mole percent of at least one crosslinker, and the atmosphere in the vessel optionally contains no more than 2 percent oxygen.

Optionally, in one variant, the bead includes at least one vinyl monomer having at least 90 mole percent styrene. The crosslinker comprises divinylbenzene, and the bead has a diameter no greater than 200 μm..

**T-20 Example**

This Example describes the preparation of a nine amino acid fragment of the peptide known as T-20, described in U.S. Pat. No. 6,015,881, Table 1, as Peptide No. 7, containing amino acids 18-35. U.S. Pat. No. 6,015,881 is incorporated herein by reference as if fully set forth. The kinetics of the reaction are followed by sampling resin periodically during the coupling and running a Kaiser test to determine the presence of any unreacted primary amine. The resins described above are used in the creation of the loaded resin, and then in the peptide build. The examples below show exemplary peptide builds. The results of this example are shown in table 4. It is appreciated that various combinations of peptide builds can be constructed using the techniques described herein.

It is appreciated that the methods described herein can be used for very low cost and efficient synthesis of peptides, in particular T-20, and T-20-like peptides. Such methods utilize solid and liquid phase synthesis procedures to synthesize and combine groups of specific peptide fragments to yield the peptide of interest. In other variant, individual peptide fragments which act as intermediates in the synthesis of the peptides of interest (e.g., T-20) are also created. In yet another aspect the present invention provides for the creation of groups of such peptide intermediate fragments which can be utilized together to produce full length T-20 and T-20-like peptides. One of ordinary skill in the art will appreciate that the cycle times for producing peptides, including but not limited to T-20 which include assembly of many smaller fragments, are in the aggregate also substantially reduced. Not only are cycle times be reduced but waste is greatly reduced, and efficiency is greatly increased.

In another aspect, the peptides or fragments of peptides created by the processes described herein are purified, and/or the individual peptide fragments which act as intermediates in the synthesis of the subject peptides are also purified.

It is further appreciated that the invention can also be used to create peptides and peptide fragments which exhibit an ability to inhibit fusion-associated events, and, importantly, also exhibit potent antiviral activity. These peptides and peptide fragments are described in U.S. Pat. Nos. 5,464,933; 5,656,480 and PCT Publication No. WO 96/19495, incorporated by reference herein as expressly set forth. The invention provides a method for creating these therapeutics in large scale quantities.

T-20 and T-20 fragments are made using solid and liquid phase synthesis procedures to synthesize and combine groups of specific peptide fragments to yield the peptide of interest. Generally, the methods of the invention include synthesizing specific side-chain protected peptide fragment intermediates of T-20 or a T-20-like peptide on a solid support created by the invention described herein, coupling the protected fragments in solution to form a protected T-20 or T-20-like peptide, followed by deprotection of the side chains to yield the final T-20 or T-20-like peptide. A preferred embodiment of the methods of the invention involves the synthesis of a T-20 peptide having an amino acid sequence as depicted in U.S. Patent No. 6,015,881 ("'881 Patent").

The present invention further relates to individual peptide fragments which act as intermediates in the synthesis of the peptides of interest (e.g., T-20). The peptide fragments of the invention include, but are not limited to, those having amino acid sequences as described in the '881 Patent.

It is appreciated that the present invention can also create one or more peptide fragments using conventional techniques using CTC-resins, and create one or more peptide fragments using the techniques described herein using the alcohol based resins as described in our patent application filed February 12, 2003 by Bohling et al., entitled "AMINO ACID LOADED TRITYL ALCOHOL RESINS, METHOD OF PRODUCTION OF AMINO ACID LOADED TRITYL ALCOHOL RESINS, AND BIOLOGICALLY ACTIVE SUBSTANCES AND THERAPEUTICS PRODUCED THEREWITH" docket no. DN A01485. The resulting peptides can thereafter be combined to obtain the T-20 peptides or T-20 like peptides.

It will be understood that the methods, fragments and groups of fragments and techniques utilized for choosing the fragments and groups of fragments of the present invention may be used to synthesize T-20-like fragments in addition to T-20. The term "T-20-like" as used herein means any HIV or non-HIV peptide listed in U.S. Pat. Nos. 5,464,933; 5,656,480 or PCT Publication No. WO 96/19495, each of which is hereby incorporated by reference in its entirety.

In addition to T-20 and the T-20-like peptides described above, the methods, fragments and groups of fragments of the present invention may be used to synthesize peptides having modified amino and/or carboxyl terminal ends.

In a preferred embodiment, the methods of the invention are used to synthesize the peptide having a formula wherein X is an acetyl group and Z is an amide group. In a preferred method, T-20 and T-20-like peptides and intermediates can be purified using any non-silica based column packing (for maximization of loading capacity) including but not limited to zirconium-based packings, poly-styrene, poly-acrylic or other polymer based packings which are stable at high (greater than >7) pH ranges. For example, among the non-silica-laded column packing exhibiting a broad pH range that includes pH valves greater than that are sold by Tosohaus (Montgomeryville, Pa.). Columns packed with such material can be run in low, medium or high pressure chromatography

The present invention also provides for large scale efficient production of peptide fragment intermediates of T-20 and T-20-like peptides with specific amino acid sequences as listed in Table 1 above of the '881 Patent, and the groups of peptide fragment intermediates listed in Table 2 of the '881 Patent. Such peptide intermediates, especially in groups as listed in Table 2 of the '881 Patent are utilized to produce T-20 and T-20 like peptides.

Any one or more of the side-chains of the amino acid residues of peptide fragments may be protected with standard protecting groups such as t-butyl (t-Bu), trityl (trt) and t-butyloxycarbonyl (Boc). The t-Bu group is the preferred side-chain protecting group for amino acid residues Tyr(Y), Thr(T), Ser(S) and Asp(D); the trt group is the preferred side-chain protecting group for amino acid residues His(H), Gln(Q) and Asn(N); and the Boc group is the preferred side-chain protecting group for amino acid residues Lys(K) and Trp(W).

During the synthesis of fragments, the side-chain of the histidine residue is be protected, preferably with a trityl (trt) protecting group. If it is not protected, the acid used to cleave the peptide fragment from the resin may detrimentally react with the histidine residue, causing degradation of the peptide fragment.

The glutamine residues of the peptide fragments of the invention are protected with trityl (trt) groups. However, it is possible not to protect the glutamine residue at the carboxy-terminal end of certain fragments. All the asparagine residues of each peptide fragment of the invention can be protected. In addition, the tryptophan residue is protected with a Boc group. Some of the individual peptide fragments are made using solid phase synthesis techniques described herein, while other peptides of the invention are optionally made using a combination of solid phase and solution phase synthesis techniques. The peptides of the invention may alternatively be synthesized such that one or more of the bonds which link the amino acid residues of the peptides are non-peptide bonds. These alternative non-peptide bonds may be formed by utilizing reactions well known to those in the art, and may include, but are not limited to imino, ester, hydrazide, semicarbazide, and azo bonds, to name but a few.

In yet another embodiment of the invention, T-20 and T-20 like peptides comprising the sequences described above may be synthesized with additional chemical groups present at their amino and/or carboxy termini, such that, for example, the stability, reactivity and/or solubility of the peptides is enhanced. For example, hydrophobic groups such as carbobenzoxyl, dansyl, acetyl or t-butyloxycarbonyl groups, may be added to the peptides' amino termini. Likewise, an acetyl group or a 9-fluorenylmethoxy-carbonyl group may be placed at the peptides' amino termini. Additionally, the hydrophobic group, t-butyloxycarbonyl, or an amido group may be added to the peptides' carboxy termini. Similarly, a para-nitrobenzyl ester group may be placed at the peptides' carboxy termini.

Further, T-20 and T-20-like peptides may be synthesized such that their steric configuration is altered. For example, the D-isomer of one or more of the amino acid residues of the peptide may be used, rather than the usual L-isomer.

Still further, at least one of the amino acid residues of the peptides of the invention may be substituted by one of the well known non-naturally occurring amino acid residues. Alterations such as these may serve to increase the stability, reactivity and/or solubility of the peptides of the invention.

Preferably, one or more the peptide fragments of the present invention are synthesized by solid phase peptide synthesis (SPPS) techniques described herein using standard FMOC protocols. See, e.g., Carpino et al., 1970, J. Am. Chem. Soc. 92(19):5748-5749; Carpino et al., 1972, J. Org. Chem. 37(22):3404-3409.

General procedures for production and loading of resins using conventional techniques can be used in addition to, or in combination with, the novel techniques described herein. Some fragments can be made using resin loading performed, for example, via the following techniques: The resin, preferably a super acid sensitive resin such as 2-chlorotrityl resin, is charged to the reaction chamber. The resin is washed with a chlorinated solvent such as dichloromethane (DCM). The bed is drained and a solution of 1.5 equivalents of an amino acid and 2.7 equivalents of diisopropylethylamine (DIEA) in about 8-10 volumes of dichloroethane (DCE) is added. The N-terminus of the amino acid should be protected, preferably with Fmoc, and the side chain of the amino acid should be protected where necessary or appropriate. The mixture is agitated with nitrogen bubbling for 2 hours. After agitation, the bed is drained and washed with DCM. The active sites on the resin are endcapped with a 9:1 MeOH:DIEA solution for about 20-30 minutes. The bed is drained, washed 4 times. with DCM and dried with a nitrogen purge to give the loaded resin.. The fragment is then built following standard washing, deprotecting, coupling and cleaving protocols. Other fragments are made using the novel techniques described herein. The fragments made by the various techniques are then combined as described.

Fmoc is the preferred protecting group for the N-terminus of the amino acid. Depending on which amino acid is being loaded, its side chain may or may not be protected. For example, when Trp is loaded, its side chain should be protected with Boc. Similarly, the side-chain of Gln may be protected with trt. However, when Gln is being loaded in preparation for the synthesis of the 1-16 peptide fragment, its side chain should not be protected. It is not necessary to protect the side-chain of Leu.

The Fmoc-protected amino acids used in loading the resin and in peptide synthesis are available, with or without side-chain protecting groups as required, from Senn or Genzyme. Other exemplary peptides and fragments described in U.S. Patent No. 6,281,331 (incorporated by reference herein as if fully set forth) can be made using the novel techniques described herein, alone or in combination with other conventional techniques.

### T-1249 Example

The processes and substrates described herein can also be used to construct the polypeptide described in U.S. Patent No. 6,469,136 ("'136 Patent"), incorporated herein by reference as if fully set forth. In particular, peptides referred to herein as T-1249 and T-1249-like peptides can be constructed using the novel methods described herein, alone or in combination with the conventional methods described herein. These methods utilize solid and liquid phase synthesis procedures to synthesize and combine groups of specific peptide fragments to yield the peptide of interest.

Novel methods for the synthesis of peptides, in particular peptides referred to herein as T-1249 and T-1249-like peptides, are described herein. These methods utilize solid and liquid phase synthesis procedures to synthesize and combine groups of specific peptide fragments to yield the peptide of interest. Generally, the methods include synthesizing specific side-chain protected peptide fragment intermediates of T-1249 or a T-1249-like peptide on a solid support, coupling the protected fragments in solution to form a protected T-1249 or T-1249-like peptide, followed by deprotection of the side chains to yield the final T-1249 or T-1249-like peptide. A preferred embodiment of the methods of the invention involves the synthesis of a T-1249 peptide having an amino acid sequence as depicted in the '136 Patent.

The present invention further provides a low cost, highly efficient method to construct individual peptide fragments which act as intermediates in the synthesis of the peptides of interest (e.g., T-1249). The peptide fragments of the invention include, but are not limited to, those having amino acid sequences as depicted in Table 1 of the '136 Patent. Combinations of solid phase liquid phase synthetic reactions as described herein allow high purity T-1249 and T-1249-like peptides to be manufactured for on a large scale with higher throughput and higher yield than those described in the art. T-1249 and T-1249-like peptides may be synthesized on a scale of one or more kilograms.

Creation of Full-length Peptides

The present invention is used to synthesize the peptide known as T-1249. T-1249 is a 39 amino acid residue polypeptide whose sequence is derived from HIV-1, HIV-2 and SIV gp41 viral polypeptide sequences. It will be understood that the methods, fragments and groups of fragments and techniques utilized for choosing the fragments and groups of fragments of the present invention may be used to synthesize T-1249-like fragments in addition to T-1249. The term "T-1249-like" as used herein means any HIV or non-HIV peptide listed in International Application No. PCT/US99/11219, filed May 20, 1999, International Publication No. WO 99/59615 published Nov. 25, 1999, which is hereby incorporated by reference in its entirety.

In addition to T-1249 and the T-1249-like peptides described above, the methods, fragments and groups of fragments of the present invention may be used to synthesize peptides having modified amino and/or carboxyl terminal ends. or other polymer based packings which are stable at high and low pH ranges.

Peptide Intermediates

One or more peptide fragment intermediates of T-1249 and T-1249-like peptides with specific amino acid sequences as listed in Table 1 of the '136 Patent, and one or more groups of peptide fragment intermediates listed in Table 2 of the '136 Patent are also constructed using the novel processes described herein, alone or in combination with other art processes.

Peptide Synthesis

Individual peptide fragments are preferably made using solid phase synthesis techniques, while other peptides of the invention are optionally made using a combination of solid phase and solution phase synthesis techniques. The syntheses culminate in the production of T-1249 or T-1249-like peptides.

The peptides of the invention may alternatively be synthesized such that one or more of the bonds which link the amino acid residues of the peptides are non-peptide bonds. These alternative non-peptide bonds may be formed by utilizing reactions well known to those in the art, and may include, but are not limited to imino, ester, hydrazide, semicarbazide, and azo bonds, to name but a few. Further, T-1249 and T-1249-like peptides may be synthesized such that their steric configuration is altered. For example, the D-isomer of one or more of the amino acid residues of the peptide may be used, rather than the usual L-isomer.

Still further, at least one of the amino acid residues of the peptides of the invention may be substituted by one of the well known non-naturally occurring amino acid residues. Alterations such as these may serve to increase the stability, reactivity and/or solubility of the peptides of the invention. Any of the T-1249 or T-1249-like peptides may be synthesized to additionally have a macromolecular carrier group covalently attached to its amino and/or carboxy termini. Such macromolecular carrier groups may include, for example, lipid-fatty acid conjugates, polyethylene glycol, carbohydrates or additional peptides.

Amino acid loaded resins are prepared using the novel techniques described herein. After agitation, the bed is drained and washed with DCM.. The bed is drained, washed four times with DCM and dried with a nitrogen purge to give the loaded resin.

Fmoc is the preferred protecting group for the N-terminus of the amino acid. Depending on which amino acid is being loaded, its side chain may or may not be protected. For example, when tryptophan (Trp) is loaded, its side chain should be protected with Boc. However, it is not necessary to protect the side-chain of leucine (Leu). Preferably, glutamic acid (Glu), aspartic acid (Asp), threonine (Thr) and serine (Ser) are protected as t-butyl ethers or t-butyl esters, and tryptophan (Trp) and lysine (Lys) are protected as t-butoxycarbonyl carbamates (Boc). The amide side-chain of asparagine (Asn) and glutamine (Gln) may or may not be protected with trityl groups.

Meanwhile, the subsequent amino acid in the sequence to be added to the resin is activated for reaction at its carboxy terminus. The amine terminus of each amino acid should be protected with Fmoc. Depending on which amino acid is being added, its side chain may or may not be protected. Preferably, the side-chains of tyr(Y), Thr(T), Ser(S), Glu(E) and Asp(P) are protected with t-Bu, the side-chains of Gln(Q) and Asn(N) are protected with trt, and the side-chains of Lys(K) and Trp(w) are protected with Boc. It is not necessary for the side-chains of Leu or lie to be protected.

The amino acid can be activated as follows. The Fmoc-protected amino acid (1.5 eq), 1-hydroxybenzotriazole hydrate (HOBT) (1.5 eq), and diisopropyl-ethylamine (DIEA) (1.5 eq) are dissolved in a polar, aprotic solvent such as N-methyl pyrrolidinone (NMP), dimethyl formamide (DMF) or dimethyl acetamide (DMAC) (about 7.5 vol.) at room temperature. The solution is chilled to 0-5 degrees C and then O-benzotriazol-1-yl-N,N,N',N'-tetramethyluronium hexafluorophosphate (HBTU) or O-benzotriazol-1-yl-tetramethyltetrafluoroborate (TBTU)(1.5 eq) is added followed by stirring for 5-15 minutes to dissolve. It is important that activation is carried out at 0-5 degrees C to minimize racemization of the amino acid. The HBTU is the last reagent added to the cold solution since activation and racemization cannot take place in its absence.

The solution of activated amino acid is charged to the drained resin, washing in with DCM (approximately 2.5 vol). Note that activation of the amino acid is carried out in NMP due to the insolubility of HBTU in DCM. However, DCM is added to the reaction at this point to maintain adequate swelling of the resin beads. The reaction is agitated with N.sub.2 bubbling for about 1 hour at 20-30 degrees. C.

If the resin is to be stored overnight between coupling cycles, the resin bed may be drained and covered with NMP under a nitrogen blanket. Alternatively, the bed may be drained, stored under a nitrogen blanket, then conditioned with a DCM wash prior to proceeding with the next coupling cycle. If the completed fragment is to be stored overnight prior to cleavage, the resin bed should be washed free of NMP with IPA because significant Fmoc deprotection can occur in NMP.

After the coupling is judged complete, the resin is drained and washed with 3 aliquots (approximately 10 vol.) of NMP. The cycle is repeated for subsequent mers (i.e., amino acids) of the peptide fragment. Following the final coupling reaction, the resin is washed with 4 aliquots (about 10 vol.) of NMP, then with 2 aliquots (approximately 10 vol.) of DCM and 2 IPA. The resin-bound peptide may be dried with a nitrogen purge or in an oven.

Peptides synthesized via solid phase synthesis techniques can be cleaved and isolated according to, for example, the following non-limiting techniques: The peptide may be cleaved from the resin using techniques well known to those skilled in the art. For example, solutions of 1% or 2% trifluoroacetic acid (TFA) in DCM or a combination of a 1% and a 2% solution of TFA in DCM may be used to cleave the peptide. Acetic acid (HOAC), hydrochloric acid (HCl) or formic acid may also be used to cleave the peptide. The specific cleavage reagent, solvents and time required for cleavage will depend on the particular peptide being cleaved. After cleavage the cleavage fractions are subjected to standard work-up procedures to isolate the peptide. Typically, the combined cleavage fractions are concentrated under vacuum, followed by reconstitution with polar aprotic or polar aprotic solvents such as ethanol (EtOH), methanol (MeOH), isopropyl alcohol (IPA), acetone, acetonitrile (ACN), dimethyl formamide (DMF), NMD, DMAC, DCM, etc., followed by precipitation or crystallization with antisolvent such as water or hexanes, and collection by vacuum filtration. Alternatively, the product may be triturated with organic solvents or water after isolation of the peptide.

For synthesis of full length T-1249 peptides, the peptide intermediates, can be coupled together to yield the T-1249 peptide. For example, the groups of peptide intermediates, above, can be coupled together to produce T-1249 full-length peptide using the one or more of the methods described herein.

In certain embodiments, a three fragment approach for synthesis of T-1249 can be followed. A "three fragment approach" synthesis refers to a T-1249 synthesis scheme which begins with three T-1249 intermediate peptide fragments that are synthesized and coupled using solid and liquid phase synthesis techniques into a full-length T-1249 peptide.

Method for Solid Phase Peptide Synthesis (SPPS); General Procedure

A SPPS chamber is charged FmocLeu-resin (1 eq). The resin is conditioned in 5% piperidine DCM (7.5 vol) with a nitrogen purge for 15-30 minutes. The solvent is drained and the resin is treated with 2.times.20% piperidine in NMP (5 volumes) for 30 minutes to remove the Fmoc protecting group. After the second 20% piperidine/NMP treatment, the resin is washed with 5-7.times.NMP (5 vol) to a negative choranil test.

Meanwhile, the subsequent amino acid (1.5 eq), HOBT (1.5 eq) and DIEA (1.5 eq) are combined in 3:1 NMP/DCM (10 vol), allowed to fully dissolve at room temperature and cooled to 0 degrees C. HBTU is added, the solution is stirred for 10-15 minutes to dissolve the solid then added to the resin. The suspension is agitated with stirring under a nitrogen atmosphere for 1-3 hours. Coupling completion is monitored with a qualitative ninhydrin test. If the reaction is incomplete after 3 h (positive ninhydrin test persists) the reactor should be drained and a recoupling should be performed with a fresh solution of activated amino acid (0.5 eq). Normally after 30 min-1 h of recoupling a negative ninhydrin test is obtained. This cycle is repeated for the remaining amino acids in the fragment. As the fragment builds, the solvent volumes used in the washes may need to be increased from 5 volumes. Following the final coupling, the resin is washed with 3.times.5-8 volumes of NMP then 2.times. 10 volumes of DCM and dried to constant weight in a vacuum oven at 40 degrees C.

Preferred Methods for Cleavage of the Peptide from Resin

The methods below describe the cleavage of peptide AcAAl-12OH from the resin. However, the same methods may be used for cleavage of other peptide fragments of the present invention.

Method A: Use of HOAc

The resin (1 g, 0.370 mmol) was treated with mixture of AcOH/MeOH/DCM (5:1:4, 20 vol, 20 mL) with nitrogen agitation for 1.5 h and the solution was transferred to a round bottom flask, stirred, and treated with water (20 vol). The resulting white slurry was concentrated (rotavap, 40 degrees. C bath) to remove DCM and the product collected by filtration. Drying to a constant weight affords 0.69 g (74%) of AcAAl-12OH in 87A % purity. A second treatment of the resin as above provided an additional 0.08 g (8.5%) of AcAAl-12OH of less pure material (83 Area %) suggesting a desired reaction time of slightly > 1.5 hr.

Method B: Use of TFA

The resin (1 wt., 20 g) is washed with 5-6.times. 1.7 volumes of 1 % TFA in DCM, 3-5 minutes each. The 1 % TFA/DCM washes are collected in a flask containing pyridine (1: 1 volume ratio with the TFA in the wash). The product containing washes are combined (600 mL, 30 vol) and the DCM is removed by distillation to a minimum pot volume (.about. 1/3 the original volume). The vacuum is adjusted to maintain a pot temperature of 15-25 degrees C. Ethanol (6.5 vol) is added and the distillation is continued until the DCM is removed (as determined by an increase in the temperature of the distillate). Again the vacuum is adjusted to maintain a pot temperature of 15-20 degrees C. The final pot volume should be .about.8-9 volumes. The solution is cooled to 5-10 degrees C and water (6.5 vol) is added over 30 minutes to precipitate the AcAA1-12OH. The solid is collected by vacuum filtration and washes with water (2-3 vol). The slurry is stirred at 0-5 degrees. C for 30 minutes, the solids are collected by vacuum filtration and dried to constant weight to give 16.80 g of AcAA1-120H in 90% yield and 84 Area % (A %) purity.

SPPS of FmocAA27-38OH and Cleavage from the Resin

SPPS of FmocAA27-38OH was carried out as described above starting with 10 g of FmocTrp(Boc)OR loaded at 0.75 mmol/g. Cleavage method B was used (169/120/1, 78% yield, 87.9A %).

HPLC Conditions: Vydac C8, cat. No. 208TP54, 5 u, 300 A, 0.9 mL/min., 280 nm. A: 0.1% TFA/water, B: A mixture of 80% I-PrOH/20% Acetonitrile and 0.1% TFA. 60-80% B/30 min. Typical sample preparation: Dissolve 1 mg in 0.10 mL NMP, dilute with 1 mL Acetonitrile. Inject 20 .mu.L into a 20 .mu.L loop.

## Reduced Use of AA Equivalents Example

The process described herein has the unexpected resultusing less reagent that convention methods. It is appreciated that the cost of reagents including various amino acids is high. The present invention provides for significantly less reagent usage than convention techniques and therefor provides significant cost savings at scale up. In one variant, less than about 1.5 equivalents of the amino acid are used per equivalent of growing peptide chain. In one variant, less than about 1.4 equivalents of the amino acid are used per equivalent of growing peptide chain. In yet another variant, less than about 1.3 equivalents of the amino acid are used per equivalent of growing peptide chain. In yet a different variant, less than about 1.2 equivalents of the amino acid are used per equivalent of growing peptide chain. In yet a further aspect, less than about 1.1 equivalents of the amino acid are used per equivalent of growing peptide chain.

The loading efficiency of the current invention was compared with that found in the '881 patent by loading the resin of the current invention with the amino acid following the process of the '881 patent. The amount of amino acid charged was compared to the amount loaded and the efficiency calculated.

Table

| Loading efficiency for several amino acids. | | |
|---|---|---|
| Leu | R+H | 89.4% |
| | '881 patent | 71.7% |
| Trp(Boc) | R+H | 66.5% |
| | '881 patent | 47.1 % |
| Gln | R+H | 82.2% |
| | '881 patent | 63.8% |

For each of the three amino acids the loading efficiency was increased substantially using the present invention as compared with that described in the '881 patent. The reduced coupling times and increased loading efficiencies can also be correlated to reduced reagent usage during the formation of the peptide fragments. The reduced reagent usage at commercial scale results in significant cost savings and reagent use.

## Reduced Re-Coupling Example

Another unexpected result of the process used herein is the fact that re-couples are greatly reduced or eliminated all together. Conventional methods result in recouples for amino acid fragments greater than about 9 amino acids. The process used herein had the unexpected result that T-20 or T-1249 fragments having greater than about 10 amino acids did not require recouples. At scale, this a further significant advantage of the process described herein, and results in significant re-work savings. In another aspect, T-20 or T-1249 fragments having greater than about 15 amino acids were produced without recouples.

## Reduced Cycle Time Example

It was further determined that the present invention provides significantly reduced cycle times over conventional methods. At scale, this feature permits capacity limited facilities to reduce the cycle time for aa loads and peptide builds. This permits a production facility with set capability to increase throughput per cycle or shift. By way of example, cycle times can be reduced by as much as 50% over conventional methods. In one variant of the invention, a cycle time reduction in the range of about 15 minutes to about 30 minutes per cycle is accomplished. Where large peptide fragments are being synthesized, e.g. a decamer, the cycle time can be reduced by anywhere from 150 minutes to 300 minutes. It is appreciated that the cycle time savings in substantial where even larger peptides are being synthesized.

Table

| Fmoc-L-AA-OH | Coupling Cycle Time (min) | |
|---|---|---|
| | R+H CTC | |
| Asn (trt) | 1 | |
| Typ (Boc) | 30 | |
| Leu | 15 | |
| Ser(t-Bu) | 30 | |
| Ala | 15 | |
| Typ (Boc) | 30 | |
| Lys (Boc) | 30 | |
| Asp (t-Bu) | 30 | |
| **Total Time** | **195** | |
| **AA Coupling** | **100%** | |
| | | |

## Recycling Example

The resin described in this invention is recycled by removing the peptide using standard conditions, then converted to the chlorotrityl alcohol resin with sodium hydroxide. The chlorotrityl alcohol resin is then converted to CTC by treatment with thionyl chloride and a catalytic amount of dimethyl formamide in toluene. The increased durability of the low void space resin has lead to significantly improved perfect bead count and processability for the recycled resin compared to the resins currently found in the art.

## Amino Acid Yield Comparison Example

The process of claim 1 further comprising obtaining a load efficiency of amino acid greater than about 75% for 1eq (per gram of CTC) FMOC-Leu and 1.35 eq (per molar eq of FMOC Leu) of diisopropylethyl amine in 10 mL dichlormethane/gram of CTC resin or 60% for 1eq (per gram of CTC) FMOC-Trp(boc) and 1.35 eq (per molar eq of FMOC-Trp(boc)) of diisopropylethyl amine in 10 mL dichlormethane/gram of CTC resin and 75% for 1eq (per gram of CTC) FMOC-Gln and 1.35 eq (per molar eq of FMOC Gln) of diisopropylethyl amine in 10 mL dichlormethane/gram of CTC resin.

While only a few, preferred embodiments of the invention have been described hereinabove, those of ordinary skill in the art will recognize that the embodiment may be modified and altered without departing from the central spirit and scope of the invention. Thus, the preferred embodiment described hereinabove is to be considered in all respects as illustrative and not restrictive, the scope of the invention being indicated by the appended claims, rather than by the foregoing description, and all changes which come within the meaning and range of equivalency of the claims are intended to be embraced herein.

## Claims

1.  An improved process for making a T-20 or a T-1249 composition, or a fragment of a T-20 or a T-1249 composition, using a chlorotrityl chloride linker-resin, in which said improvement comprises:

    using a low void space resin optionally loaded with an amino acid or amino acid derivative to create one or more T-20 or T-1249 fragments.

2.  The process of claim 1 in which less than 1.5 equivalents of said amino acid are used per equivalent of growing peptide chain.

3. The process of claim 2 further comprising providing a cycle time reduction in the range of about 15 minutes to about 30 minutes over conventional methods.

4. The process of claim 1 further comprising obtaining a load efficiency of amino acid greater than about a 7.5% increase over conventional resins.

5. The process of claim 1 further comprising recycling said low void space resin.

6. The process of claim 1 further comprising preparing a T-20 or T-1249 fragment having greater than about 10 amino acids, said process being free of recouples.

7. The process of claim 1 further comprising preparing a T-20 or T-1249 fragment having greater than about 15 amino acids, said process being free of recouples.

8. The process of claim 1 further comprising recycling said low void space resin.

9. A T-20 or T-1249 composition, in which one or more fragments of T-20 or T-1249 are made by the process of claim 1.

10. A improved process for making a T-20 or T-1249 composition, a fragment of a T-20 or T-1249 composition using a chlorotrityl chloride linker-resin, in which said improvement comprises:

   using a resin having functionality biased toward the surface of said resin, and said resin being optionally loaded with an amino acid or amino acid derivative, to create one or more T-20 or T-1249 fragments.

11. The process of claim 10 further comprising recycling said resin.

12. A T-20 or T-1249 composition, in which one or more fragments of T-20 or T-1249 are made by the process of claim 10.

13. A improved process for making a T-20 or T-1249 composition, a fragment of a T-20 or T-1249 composition using a chlorotrityl chloride linker-resin, in which said improvement comprises:

   using a low void space resin having functionality biased toward the surface of said low void space resin, and said low void space resin being optionally loaded with an amino acid or amino acid derivative, to create one or more T-20 or T-1249 fragments.

14. A T-20 or T-1249 composition, in which one or more fragments of T-20 or T-1249 are made by the process of claim 13.

**European Patent Office**

# EUROPEAN SEARCH REPORT

Application Number

EP 04 25 0807

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | US 6 015 881 A (BRAY BRIAN ET AL) 18 January 2000 (2000-01-18) Whole document and in particular column 10, last paragraph and Example 6. | 1-14 | C07K1/04 C07K17/06 |
| Y | * the whole document * | 1-14 | |
| X,D | US 6 469 136 B1 (FRIEDRICH PAUL ERICKSON ET AL) 22 October 2002 (2002-10-22) Whole document and in particular column 8, lines 25-36 and Example 6. | 1-14 | |
| Y | * the whole document * | 1-14 | |
| Y | RANA S ET AL: "Influence of resin cross-linking on solid-phase chemistry." JOURNAL OF COMBINATORIAL CHEMISTRY. UNITED STATES 2001 JAN-FEB, vol. 3, no. 1, January 2001 (2001-01), pages 9-15, XP002285867 ISSN: 1520-4766 Whole document and in particular Abstract, page 10, last paragraph and Conclusions. | 1-14 | |
| E | EP 1 403 290 A (ROHM & HAAS) 31 March 2004 (2004-03-31) * example 8 * | 1-14 | TECHNICAL FIELDS SEARCHED (Int.Cl.7) C07K |
| A | WO 01/85758 A (MEININGHAUS CARSTEN ;LONZA AG (CH)) 15 November 2001 (2001-11-15) | | |
| A | PATENT ABSTRACTS OF JAPAN vol. 1996, no. 12, 26 December 1996 (1996-12-26) & JP 08 217794 A (BIO KOSUMOSU:KK), 27 August 1996 (1996-08-27) * abstract * | | |
| A | DE 43 06 839 A (BAYER ERNST PROF DR) 8 September 1994 (1994-09-08) | | |

The present search report has been drawn up for all claims

| Place of search | Date of completion of the search | Examiner |
|---|---|---|
| MUNICH | 24 June 2004 | Ury, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                EP 04 25 0807

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2004

| Patent document cited in search report | | Publication date | Patent family member(s) | | Publication date |
|---|---|---|---|---|---|
| US 6015881 | A | 18-01-2000 | US | 6281331 B1 | 28-08-2001 |
| | | | AU | 751358 B2 | 15-08-2002 |
| | | | AU | 3109799 A | 18-10-1999 |
| | | | BR | 9909018 A | 16-10-2001 |
| | | | CA | 2324348 A1 | 30-09-1999 |
| | | | CN | 1303299 T | 11-07-2001 |
| | | | EP | 1071442 A1 | 31-01-2001 |
| | | | JP | 2002507576 T | 12-03-2002 |
| | | | NZ | 507083 A | 31-10-2003 |
| | | | WO | 9948513 A1 | 30-09-1999 |
| US 6469136 | B1 | 22-10-2002 | AU | 4133701 A | 06-06-2001 |
| | | | BR | 0012249 A | 11-11-2003 |
| | | | CA | 2378086 A1 | 17-05-2001 |
| | | | CN | 1450906 T | 22-10-2003 |
| | | | EP | 1372686 A2 | 02-01-2004 |
| | | | JP | 2003530311 T | 14-10-2003 |
| | | | WO | 0134635 A2 | 17-05-2001 |
| | | | US | 2003125516 A1 | 03-07-2003 |
| EP 1403290 | A | 31-03-2004 | EP | 1403290 A1 | 31-03-2004 |
| | | | US | 2004063856 A1 | 01-04-2004 |
| WO 0185758 | A | 15-11-2001 | AU | 7842401 A | 20-11-2001 |
| | | | WO | 0185758 A2 | 15-11-2001 |
| | | | EP | 1280831 A2 | 05-02-2003 |
| | | | US | 2003092847 A1 | 15-05-2003 |
| JP 08217794 | A | 27-08-1996 | NONE | | |
| DE 4306839 | A | 08-09-1994 | DE | 4306839 A1 | 08-09-1994 |

EPO FORM P0459

For more details about this annex : see Official Journal of the European Patent Office, No. 12/82